(19) Europäisches Patentamt
European Patent Office
Office européen des brevets

(11) **EP 4 200 319 B1**

(12) **EUROPEAN PATENT SPECIFICATION**

(45) Date of publication and mention of the grant of the patent:
**16.04.2025 Bulletin 2025/16**

(21) Application number: **21765917.6**

(22) Date of filing: **20.08.2021**

(51) International Patent Classification (IPC):
*C07K 14/47* (2006.01)      *A61K 38/17* (2006.01)
*A61K 47/26* (2006.01)      *A61K 47/10* (2017.01)
*A61K 9/19* (2006.01)      *A61K 47/02* (2006.01)
*A61K 9/00* (2006.01)

(52) Cooperative Patent Classification (CPC):
**C07K 14/4721; A61K 9/0019; A61K 9/19; A61K 38/1709; A61K 47/02; A61K 47/10; A61K 47/26**

(86) International application number:
**PCT/EP2021/073186**

(87) International publication number:
**WO 2022/038281 (24.02.2022 Gazette 2022/08)**

(54) **ANNEXIN A1 N-TERMINAL PEPTIDE FORMULATIONS AND METHODS**

ANNEXIN-A1-N-TERMINALE PEPTIDFORMULIERUNGEN UND VERFAHREN

FORMULATIONS DE PEPTIDE N-TERMINALE ANNEXINE A1 ET PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2020 EP 20192142**

(43) Date of publication of application:
**28.06.2023 Bulletin 2023/26**

(73) Proprietor: **RESOTHER PHARMA A/S 2840 Holte (DK)**

(72) Inventors:
• **BUKRINSKI, Jens Thostrup**
  **2500 Valby (DK)**
• **TUELUNG, Pernille Sønderby**
  **2000 Frederiksberg (DK)**
• **JENSEN, Mette Hamborg**
  **3520 Farum (DK)**

(74) Representative: **Høiberg P/S**
  **Adelgade 12**
  **1304 Copenhagen K (DK)**

(56) References cited:
**WO-A2-2012/174397**

• **SHAO GANG ET AL: "Advancements of Annexin A1 in inflammation and tumorigenesis", vol. Volume 12, 1 April 2019 (2019-04-01), pages 3245 - 3254, XP055776102, Retrieved from the Internet <URL:https://www.dovepress.com/getfile.php?fileID=49436> [retrieved on 20211116], DOI: 10.2147/OTT.S202271**
• **PERRETTI MAURO ET AL: "Characterizing the anti-inflammatory and tissue protective actions of a novel Annexin A1 peptide", PLOS ONE, vol. 12, no. 4, 1 April 2017 (2017-04-01), pages e0175786, XP055862058, DOI: 10.1371/journal.pone.0175786**

Remarks:
The complete document including Reference Table(s) and the Sequence Listing(s) can be downloaded from the EPO website

## Description

### Technical field

The present invention relates to pharmaceutical formulations comprising an Annexin A1 (AnxA1) N-terminal-peptide with improved solubility and/or stability, including liquid formulations. The liquid formulations have a pH > 6, and comprises a therapeutically effective amount of an Annexin A1 (AnxA1) N-terminal-peptide and a solvent selected from the group consisting of water, such as ultrapure water; and a solution having an ionic strength of 1 $\mu$M to 50 mM; the solvent comprising an isotonicity modifier selected from the group consisting of a carbohydrate and/or a sugar alcohol. Alternatively, the liquid formulations have a pH $\geq$ 8, and comprises a therapeutically effective amount of an Annexin A1 (AnxA1) N-terminal-peptide and a solvent being a solution having an ionic strength of 1 $\mu$M to 50 mM. The disclosed formulations are particularly effective for use in the treatment of ischemic and inflammatory conditions.

### Background

The Annexin super-family consists of 13 calcium phospholipid binding proteins with significant biological and structural homology. Annexins are structurally divided into a highly conserved core domain and a variable N-terminal domain. Annexin A1 (ANXA1, 37 kDa - SEQ ID NO:1) is an anti-inflammatory protein that inhibits extravasation of blood-borne polymorphonuclear leukocyte (PMN) into the surrounding tissue. The protein binds to the N-formyl peptide receptor (FPR) 2 or FPR-L1 receptor, where it initiates a cascade of signaling events. Following an inflammatory stimulus, migration of blood-borne polymorphonuclear leukocyte (PMN) into the surrounding tissue takes place. Transmigration or extravasation of PMN is regulated by mediators such as adhesion molecules, cytokines and proteases, which control the pro-inflammatory and anti-inflammatory processes. The disruptive potential of the PMN is high and potentially self-damaging. Thus, controlling extravasation of PMN and the inflammatory response is important.

For therapeutic purposes as an anti-inflammatory agent, the full Annexin A1 protein has numerous disadvantages relative to functional fragments or modified versions thereof. The large size of the protein makes it more difficult to deliver by techniques that are possible with a smaller polypeptide (e.g. transdermally or transmucosally). For use to treat inflammation of the eyes, a smaller molecule is expected to be better able to penetrate the corneal epithelium. Also, susceptibility to proteolytic degradation is a particular concern for all peptide pharmaceuticals, especially large ones and especially if oral delivery (preferred by many patients) is contemplated.

Some Annexin A1 derivatives lacking significant regions on the N-terminal side of the polypeptide have been shown to lack significant activity in some assays of inflammation and mediator release, whereas the full length N-terminus N-acetyl Annexin A1 was deemed biologically active in several systems. A number of peptides primarily derived from the unique N-terminal portion of the Annexin A1 protein have been shown to possess anti-inflammatory properties. One of the most extensively studied Annexin A1 peptides is peptide Ac2-26, which mimics the 2nd to the 26th amino acids of the 54-amino acid N-terminal region. Like the Ac 1-188 fragment (and the native protein), it has an N-terminal acetylation to increase its stability, and possibly its half-life. It has been show that Annexin A1 and its N-terminal peptide (Ac2-26) exert the majority of their anti-inflammatory action through the FPR2/Lipoxin A4 (FPR2/Alx) receptor. In vivo the Ac2-26 peptide has been shown to exert an anti-inflammatory effect in models of myocardial ischaemia reperfusion (I/R), mesentery I/R, glycogen peritonitis and IL1 airpouch, where it was reported to significantly reduce the recruitment of neutrophils to the site of injury/inflammation.

Shorter versions of the Ac2-26 peptide, such as peptides Ac2-12 and Ac2-6, have also been shown to elicit some degree of anti-inflammatory effects in acute models of inflammation. Longer polypeptides with anti-inflammatory effects, such as polypeptides corresponding to amino acid residues 2-48, 2-50 and 11-48, have been disclosed, including protease-resistant V24L variants (WO 2012/174397).

Peptide therapeutics have the potential to self-associate, leading to aggregation and/or fibrillation. To prepare stable peptide formulations the native peptide conformation should be maintained. Stable formulations and long-term stability are important in the development of effective therapeutic compositions that retains bioavailability, and which can be used in delivery devices that expose the peptide to mechanical stress (e.g. continuous infusion systems).

The solubility of peptides in aqueous solutions is highly dependent on a number of factors including choice of buffering systems, tonicity provider and the concentration and form of the peptide (e.g. salts and other modifications).

Given the therapeutic potential of Annexin A1 (AnxA1) N-terminal-peptides there is a need for developing safe, effective and stable formulations that can be administered by various routes of administration.

### Summary

The invention is defined by the claims. Any subject matter falling outside the scope of the claims is provided for information purposes only.

Annexin A1 (AnxA1) N-terminal-peptides, such as Annexin A1 2-48 V24L (SEQ ID NO:8) are shown herein to be poorly soluble with a very high fibrillation propensity in saline at neutral pH around 7,3 and as such not suitable for pharmaceutical administration. To overcome problems with solubility and stability the present inventors have developed stable solutions of Annexin A1 N-terminal-peptides, which reduce the propensity to form fibrils and particles, and preserve the peptide during optional freeze-drying to render it stable during storage of same.

The present disclosure relates to the finding that Annexin A1 N-terminal-peptides display a clear pH-dependent fibrillation profile, being completely insoluble at pH 6 and below in any solvent tested (saline, ultrapure water and essentially non-ionic and isotonic solutions). Hence maintaining a pH above 6; such as above 6,5; and maintaining an essentially non-ionic or low ionic strength (e.g. 1 µM to 50 mM) solution, such as by avoiding the presence of salts including saline; and optionally maintaining an essentially isotonic solution; markedly and unexpectedly increases solubility and stability of said Annexin A1 N-terminal-peptides.

The pharmaceutical formulations disclosed herein with improved solubility and/or stability comprise a therapeutically effective amount of an Annexin A1 N-terminal-peptide and a solvent selected from the group consisting of water, such as ultrapure water; and a solution having an ionic strength of 1 µM to 50 mM, and optionally essentially isotonic solution, said solution optionally comprising an isotonicity modifier, such as a carbohydrate and/or a sugar alcohol, wherein said formulations maintains a pH > 6.

The pharmaceutical formulations are particularly useful in the treatment of ischemic and inflammatory conditions.

It is an aspect of the present disclosure to provide a liquid pharmaceutical formulation of pH > 6; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV
AA (SEQ ID NO:4; Annexin A1 2-50),

AMVSEFLKQA WFI EN EEQEYVQTVKSSKGGPGSA VSPYPTFN PSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV
AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent selected from the group consisting of

i. water, such as ultrapure water; and
ii. a solution having an ionic strength of 1 µM to 50 mM,

wherein said solvent comprises one or more isotonicity modifiers selected from a carbohydrate and/or a sugar alcohol.

It is another aspect of the present disclosure to provide a liquid pharmaceutical formulation of pH ≥ 8; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV
AA (SEQ ID NO:4; Annexin A1 2-50),

AMVSEFLKQA WFI EN EEQEYVQTVKSSKGGPGSA VSPYPTFN PSSOV (SEQ ID NO:5; Annexin A1 2-48),

AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV

AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L),
and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent, wherein said solvent is a solution having an ionic strength of 1 μM to 50 mM.

In one embodiment said Annexin A1 N-terminal-peptide is selected from the group consisting of:

AMVSEFLKQAWFIENEEQEYVQTVKSSKGGPGSAVSPYPTFNPSSDVAA (SEQ ID NO:4; Annexin A1 2-50),
AMVSEFLKQAWFIENEEQEYVQTVKSSKGGPGSAVSPYPTFNPSSDV (SEQ ID NO:5; Annexin A1 2-48), and
AMVSEFLKQAWFIENEEQEYVQTVKSSKGGPGSAVSPYPTFNPSS (SEQ ID NO:6; Annexin A1 2-46),
and a variant of any one of SEQ ID NOs:4-6 comprising 1 to 6 individual amino acid substitutions, including variants having a V24L substitution and C-terminal amidation, wherein said variant is a ligand and/or agonist of one or more of Formyl Peptide Receptor 1 (FPR1), Formyl Peptide Receptor 2 (FPR2) and Formyl Peptide Receptor 3 (FPR3).

It is also an aspect of the present disclosure to provide a process of preparing a lyophilized pharmaceutical formulation, the process comprising the steps of

a. providing the liquid pharmaceutical formulation according to any of the preceding claims, and
b. lyophilizing the liquid pharmaceutical formulation of step a.

It is also an aspect of the present disclosure to provide a method of making a reconstituted solution comprising an Annexin A1 N-terminal-peptide, said method comprising

a. providing a lyophilized pharmaceutical formulation according to the present disclosure, and
b. dissolving said lyophilized pharmaceutical formulation in a solvent.

## Description of Drawings

Figure 1: Results from the ThT assay indicating a reverse relationship between concentration and stability from the increase in lag-time when concentration is lower. It is seen that at 6.4 mg/mL, possibly 5.0 mg/ml, and above, the sample is already degraded by the formation of fibrils at time zero.

Figure 2: Photo of 5 mg/mL AnxA1 in Milli Q® water, 0.9% NaCl (154 mM) at pH=8; pH=6; pH=4 and; pH=2. Al solutions are opaque indicating limited solubility of AnxA1

Figure 3: Concentration (A280) as a function of pH for 3 peptide concentrations. Three samples of AnxA1 was prepared at pH 9 in MiliQ® water, 0.9 % NaCl (154 mM) at concentrations 3.3, 1.6 and 0.8 mg/mL. pH was adjusted by addition of 0.1 M NaOH and the concentration determined using UV-VIS spectroscopy. It is clearly seen that below pH 6.0 solubility is very low.

Figure 4: Graphical representation of results presented in table 2 of Example 4.

Figure 5: Photograph of the visual appearance of samples containing AnxA1 at different pH values. A clear solution was observed ≥ 7.4 and from 2.6 - pH 2.0.

Figure 6: Photograph of visual appearance of solution in Milli Q® water with and without mannitol from pH 2 to 10. A clear solution is observed at pH 8 and 10, both with and without mannitol.

Figure 7: Graphical representation of the data presented in table 5 of Example 7.

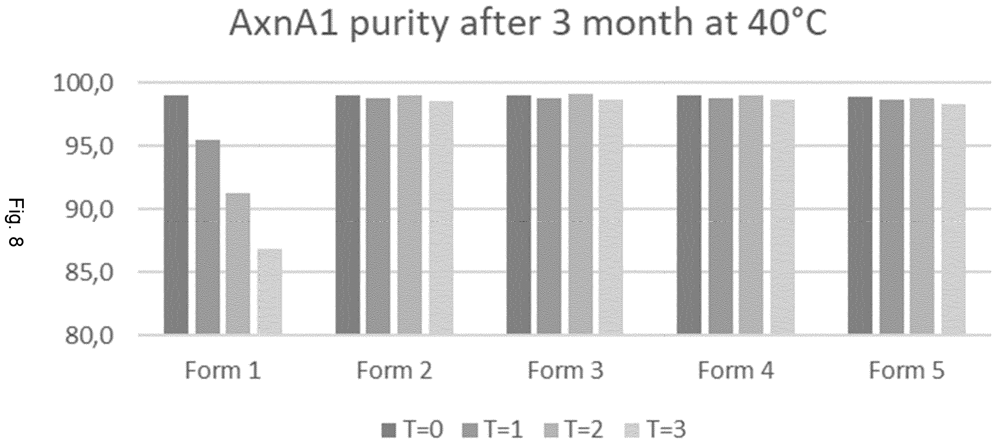

Figure 8: AnxA1 purity in reconstituted solutions after up to 3 months storage of lyophilised formulation at 40°C, with measurements conducted at 0 months (T=0), 1 month (T=1), 2 months (T=2) and 3 months (T=3).

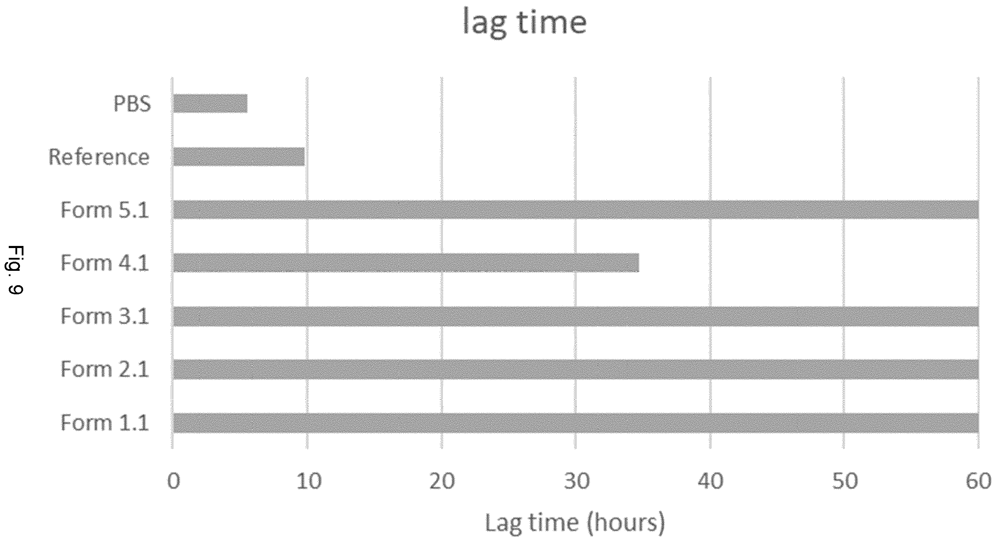

Figure 9: Graphical representation of lag times (hours) determined using the ThT assay for the formulations represented in table 9. No fibrillation is observed at pH 8.3 within analytical method (60 hours) independent of the tonicity provider, whereas at pH 7.5 the solution fibrillates after 35 hours. When comparing to the reference solution in PBS and the formulation in saline (both at pH 7.4) it is also clear that using carbohydrates as tonicity providers is advantageous.

Figure 10: Contour plot of the experimental data from example 10. Illustrating the effect of pH, AnxA1 concentration and concentration of glycyl-glycine on lag time in the ThT assay (indicated by the numbers in the white boxes and with darker colours indicating longer lag-times). From the contour plot it is seen that physical stability (long lag-time) is dependent on i.a. low concentration of glycyl-glycine, low concentration of AnxA1 and high pH.

Figure 11: Contour plot of the experimental data from example 10. Illustrating the effect of pH, AnxA1 concentration and concentration of glycyl-glycine on lag time in the ThT assay (indicated by the numbers in the white boxes and with darker colours indicating longer lag-times). From the contour plot it is seen that the physical stability (long lag-time) is dependent on i.a. low concentration of glycyl-glycine, low concentration of AnxA1 and high pH.

## *Definitions*

The singular forms "a," "an," and "the" include plural referents unless the context clearly dictates otherwise.

The term "subject" or "individual" for purposes of treatment includes any subject, and preferably is a subject who is in need of treatment of an ischemic and inflammatory condition. The subject is typically an animal, more typically a mammal. Preferably, the mammal is a human.

A "therapeutically effective amount" refers to an amount of an Annexin A1 N-terminal-peptide that is nontoxic but sufficient in treating, preventing or ameliorating the severity of an ischemic and inflammatory condition.

As used herein, "percent," "percentage" or the symbol "%" means the percent of the component indicated in the composition based on the amount of the carrier present in the composition, on a weight/weight (w/w), weight/volume (w/v) or volume/volume (v/v) concentration, as indicated with respect to any particular component, all based on the amount of the carrier present in the composition. Thus, different types of carriers can be present in an amount of up to 100% as indicated, which does not preclude the presence of the API, the amount of which can be indicated as a % or as a certain number of mg present in the composition or a certain number of mg/mL present, where the % or mg/mL is based on the amount of the total carrier present in the composition. Certain types of carriers can be present in combination to make up 100% of the carrier.

A "pharmaceutically acceptable carrier" as used herein is non-toxic or has certain toxic attributes that are not dose limiting to achieve therapeutic advantages to recipients at the dosages and concentrations required and is compatible with other ingredients of the formulation.

The term "pharmaceutically acceptable excipient," includes vehicles, adjuvants, or diluents or other auxiliary substances, such as those conventional in the art, which are readily available to the public, and which are non-toxic or have acceptable toxicities to recipients at the dosages and concentrations employed, and is compatible with other ingredients of the formulation. For example, pharmaceutically acceptable auxiliary substances include pH adjusting and buffering agents, tonicity adjusting agents, stabilizers, anti-oxidants, preservatives, solubilizes, wetting agents and the like.

The term "pharmaceutical composition" or "pharmaceutical formulation" is intended to encompass a drug product including the active ingredient(s), pharmaceutically acceptable excipients that make up the carrier, as well as any product which results, directly or indirectly, from combination, complexation or aggregation of any two or more of the ingredients. Accordingly, the pharmaceutical compositions of the present disclosure encompass any composition made by admixing the active ingredient, active ingredient dispersion or composite, additional active ingredient(s), and pharmaceutically acceptable excipients.

## Detailed description

Annexin A1 N-terminal-peptides, such as Annexin A1 2-48 V24L (SEQ ID NO:8) are poorly soluble in saline at neutral pH around 7,3 and as such not suitable for pharmaceutical administration.

On the basis of that, there is a need for developing safe, effective and stable formulations that can be administered by various routes of administration. The formulations according to the present disclosure are provided in the form of a liquid pharmaceutical formulation which are stable and also suitable for freeze-drying.

*Liquid formulation*

It is an aspect to provide a liquid formulation, such as a liquid pharmaceutical formulation, of pH > 6; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSDV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV

AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent selected from the group consisting of

iii. water, such as ultrapure water; and
iv. a solution having an ionic strength of 1 $\mu$M to 50 mM,
wherein said solvent comprises one or more isotonicity modifiers selected from a carbohydrate and/or a sugar alcohol.

It is another aspect of the present disclosure to provide a liquid pharmaceutical formulation of pH $\geq$ 8; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV

AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent, wherein said solvent is a solution having an ionic strength of 1 $\mu$M to 50 mM.

In one embodiment said solution comprises an isotonicity modifier, such as a carbohydrate and/or a sugar alcohol.

An 'isotonicity modifier' is an agent that is added to adjust the tonicity of a solution.

In one embodiment the liquid pharmaceutical formulation according to the present disclosure has improved physical stability.

In one embodiment the liquid pharmaceutical formulation comprising an Annexin A1 N-terminal-peptide according to the present disclosure has improved solubility and/or reduced insolubility.

In one embodiment said liquid pharmaceutical formulation comprising an Annexin A1 N-terminal-peptide according to the present disclosure has reduced tendency to form fibrils, such as does not form fibrils.

In one embodiment the liquid pharmaceutical formulation according to the present disclosure comprises essentially no fibrils.

In one embodiment the liquid pharmaceutical formulation according to the present disclosure has low fibrillation propensity. In one embodiment the liquid pharmaceutical formulation according to the present disclosure has a low probability of forming fibrils. In one embodiment the liquid pharmaceutical formulation according to the present disclosure has a low probability of forming fibrils upon storage.

In one embodiment the liquid pharmaceutical formulation according to the present disclosure comprises essentially no visible particles.

In one embodiment the liquid pharmaceutical formulation according to the present disclosure comprises essentially no visible and/or subvisible particles.

In one embodiment the liquid pharmaceutical formulation according to the present disclosure comprises essentially no visible and/or subvisible proteinaceous particles.

Subvisible particles are usually defined as being in the size range 0.1$\mu$m to 100$\mu$m, often above the upper size limit for measurement by size-exclusion chromatography but not sufficiently large to be readily detectable by eye. Such particles may be proteinaceous (aggregates or proteins adsorbed onto other contaminants).

In one embodiment the liquid pharmaceutical formulation according to the present disclosure comprises essentially no proteinaceous particles of $\geq$ 0,1 $\mu$m.

In one embodiment the liquid pharmaceutical formulation according to the present disclosure comprises essentially no proteinaceous particles of $\geq$ 1 $\mu$m.

In one embodiment the liquid pharmaceutical formulation according to the present disclosure comprises essentially no proteinaceous particles of $\geq$ 10 $\mu$m.

In one embodiment the liquid pharmaceutical formulation according to the present disclosure comprises essentially no proteinaceous particles of $\geq$ 100 $\mu$m.

In one embodiment the liquid pharmaceutical formulation comprising an Annexin A1 N-terminal-peptide according to the present disclosure has

a. improved physical stability,
b. improved solubility and/or reduced insolubility,
c. reduced tendency to form fibrils,
d. improved chemical stability, and/or
e. improved purity, and/or reduced impurity, and/or improved content,

as compared to a liquid pharmaceutical formulation with a pH of 3 < pH < 6; and/or
as compared to a saline liquid pharmaceutical formulation; and/or
as compared to a solution having an ionic strength of > 50 mM; and/or
as compared to a solution without an isotonicity modifier.

A solution is a homogeneous mixture composed of two or more substances. In such a mixture, a solute is a substance (such as a peptide) dissolved in another substance, known as a solvent.

pH is defined as the decimal logarithm of the reciprocal of the hydrogen ion activity, aH+, in a solution. Typically, the pH of solutions will change as temperature changes. Hence, in one embodiment, reference herein to a certain pH value is meant to indicate a pH value at room temperature and/or ambient temperature. Ambient temperature is a term which refers to the actual temperature of the air (or other medium and surroundings) in any particular place, as measured by a thermometer, or the temperature which surrounds an object. In a particular embodiment pH is measured at approx. 25 degree Celsius.

Maintaining pH at the prescribed level plays an important role in the stability of the Annexin A1 N-terminal peptides in solution. Specifically, maintaining a pH above 6 is essential in avoiding fibrillation (decreased physical stability), and the inventors have found that maintaining the pH of the pharmaceutical formulation at around 7 to 9, such as 8 - 8,5, for example around 8,3 is advantageous in some embodiments. Typical peptide formulations have a more neutral pH of around 7 - 7,8, or even an acidic pH.

*pH*

In one embodiment the liquid pharmaceutical formulation of the present disclosure has a pH > 6 at room temperature. In one embodiment said liquid pharmaceutical formulation has a pH > 6 at ambient temperature. In one embodiment said liquid pharmaceutical formulation has a pH > 6 at around 25°C.

In one embodiment the pH of the liquid pharmaceutical formulation is pH $\geq$ 6,5.

In one embodiment the pH of the liquid pharmaceutical formulation is pH $\geq$ 6,5, such as pH $\geq$ 7,0, such as pH $\geq$ 7,5, such as pH $\geq$ 8,0. In one embodiment the pH of the liquid pharmaceutical formulation is pH $\geq$ 7,4.

In one embodiment the pH of the liquid pharmaceutical formulation is pH $\geq$ 7,4, such as pH $\geq$ 7,5, such as pH $\geq$ 7,6, such as pH $\geq$ 7,7, such as pH $\geq$ 7,8, such as pH $\geq$ 7,9, such as pH $\geq$ 8,0.

In one embodiment the pH of the liquid pharmaceutical formulation is pH $\leq$ 12, such as pH $\leq$ 11,5, such as pH $\leq$ 11, such as pH $\leq$ 10,5, such as pH $\leq$ 10, such as pH $\leq$ 9,5, such as pH $\leq$ 9, such as pH $\leq$ 8,5.

In one embodiment the pH of the liquid pharmaceutical formulation is 6 > pH $\leq$ 6,5, such as 6,5 $\geq$ pH $\leq$ 7, such as 7 $\geq$ pH $\leq$ 7,5, such as 7,5 $\geq$ pH $\leq$ 8, such as 8 $\geq$ pH $\leq$ 8,5, such as 8,5 $\geq$ pH $\leq$ 9, such as 9 $\geq$ pH $\leq$ 9,5, such as 9,5 $\geq$ pH $\leq$ 10, such as 10 $\geq$ pH $\leq$ 10,5, such as 10,5 $\geq$ pH $\leq$ 11, such as 11 $\geq$ pH $\leq$ 11,5, such as 11,5 $\geq$ pH $\leq$ 12.

In one embodiment the pH of the liquid pharmaceutical formulation is 7,0 $\geq$ pH $\leq$ 7,5, such as 7,5 $\geq$ pH $\leq$ 8, such as 8 $\geq$ pH $\leq$ 8,5, such as 8,5 $\geq$ pH $\leq$ 9, such as 9 $\geq$ pH $\leq$ 9,5, such as 9,5 $\geq$ pH $\leq$ 10, such as 10 $\geq$ pH $\leq$ 10,5.

In one embodiment the pH of the liquid pharmaceutical formulation is 7,0 $\geq$ pH $\leq$ 7,5, such as 7,5 $\geq$ pH $\leq$ 8, such as 8 $\geq$ pH $\leq$ 8,5, such as 8,5 $\geq$ pH $\leq$ 9, such as 9 $\geq$ pH $\leq$ 9,5.

In one embodiment the pH of the liquid pharmaceutical formulation is such as 7,5 $\geq$ pH $\leq$ 8, such as 8 $\geq$ pH $\leq$ 8,5, such as 8,5 $\geq$ pH $\leq$ 9.

In one embodiment the pH of the liquid pharmaceutical formulation is 8 $\geq$ pH $\leq$ 8,5, such as 8,5 $\geq$ pH $\leq$ 9. In one embodiment the pH of the liquid pharmaceutical formulation is 8 $\geq$ pH $\leq$ 8,5.

In one embodiment the pH of the liquid pharmaceutical formulation is (approximately) 7,4, such as 7,5, such as 7,6, such as 7,7, such as 7,8, such as 7,9, such as 8,0, such as 8,1, such as 8,2, such as 8,3, such as 8,3, such as 8,4, such as 8,5, such as 8,6, such as 8,7, such as 8,8, such as 8,9, such as 9,0.

In one embodiment the pH of the liquid pharmaceutical formulation is 8 $\geq$ pH $\leq$ 8,5.

In one embodiment the pH of the liquid pharmaceutical formulation is approximately 8,3.

In one embodiment the pH of the liquid pharmaceutical formulation is pH $\geq$ 8.

*Solubility / Concentration*

In one embodiment the Annexin A1 N-terminal-peptide has an aqueous solubility at pH > 6, such as pH $\geq$ 6,5, such as at 6 > pH $\leq$ 6,5, such as 6,5 $\geq$ pH $\leq$ 7, such as 7 $\geq$ pH $\leq$ 7,5, such as 7,5 $\geq$ pH $\leq$ 8, such as 8 $\geq$ pH $\leq$ 8,5, such as 8,5 $\geq$ pH $\leq$ 9, such as 9 $\geq$ pH $\leq$ 9,5, such as 9,5 $\geq$ pH $\leq$ 10, of at least 1,0 mg/ml, such as of at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml.

In one embodiment the Annexin A1 N-terminal-peptide has an aqueous solubility of at least 0,1 mg/ml, such as at least 0,5 mg/ml, 1,0 mg/ml, such as at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml.

In one embodiment the Annexin A1 N-terminal-peptide has an aqueous solubility of at least 0,8 mg/ml, such as at least 1 mg/ml, such as at least 1,6, such as at least 2, such as at least 2,5, such as at least 3, such as at least 3,3, such as at least 3,5 mg/ml.

In one embodiment the liquid pharmaceutical formulation comprises said Annexin A1 N-terminal-peptide in a concentration of 0,1 to 50 mg/ml; such as in a concentration of 0,1 to 0,5 mg/ml, such as 0,5 to 1, such as 1 to 2, such as 2 to 3, such as 3 to 4, such as 4 to 5, such as 5 to 6, such as 6 to 7, such as 7 to 8, such as 8 to 9, such as 9 to 10, such as 10 to 11, such as 11 to 12, such as 12 to 13, such as 13 to 14, such as 14 to 15, such as 15 to 16, such as 16 to 17, such as 17 to 18, such as 18 to 19, such as 19 to 20 mg/ml, such as 20 to 21, such as 21 to 22, such as 22 to 23, such as 23 to 24, such as 24 to 25, such as 25 to 26, such as 26 to 27, such as 27 to 28, such as 28 to 29, such as 29 to 30 mg/ml, such as 30 to 31, such as 31 to 32, such as 32 to 33, such as 33 to 34, such as 34 to 35, such as 35 to 36, such as 36 to 37, such as 37 to 38, such as 38 to 39, such as 39 to 40 mg/ml, such as 40 to 41, such as 41 to 42, such as 42 to 43, such as 43 to 44, such as 44 to 45, such as 45 to 46, such as 46 to 47, such as 47 to 48, such as 48 to 49, such as 49 to 50 mg/ml.

In one embodiment the liquid pharmaceutical formulation comprises said Annexin A1 N-terminal-peptide in a concentration of at least 0,1 mg/ml, such as at least 0,5 mg/ml, such as 1,0 mg/ml, such as at least 1,5 mg/ml, such as at least 2,0 mg/ml, such as at least 2,2 mg/ml, such as at least 2,5 mg/ml, such as at least 3,0 mg/ml, such as at least 3,5 mg/ml, such as at least 4,0 mg/ml, such as at least 4,5 mg/ml, such as at least 5,0 mg/ml.

In one embodiment the liquid pharmaceutical formulation comprises said Annexin A1 N-terminal-peptide in a concentration of at least 0,8 mg/ml, such as at least 1 mg/ml, such as at least 1,6, such as at least 2, such as at least 2,5, such as at least 3, such as at least 3,3, such as at least 3,5 mg/ml.

In one embodiment the liquid pharmaceutical formulation has a pH >6 and the Annexin A1 N-terminal-peptide has an aqueous solubility of at least 0,8 mg/ml, such as at least 1 mg/ml, such as at least 1,6, such as at least 2, such as at least 2,5, such as at least 3, such as at least 3,3, such as at least 3,5 mg/ml; and/or said formulation has a concentration of said Annexin A1 N-terminal-peptide of at least 0,8 mg/ml, such as at least1 mg/ml, such as at least 1,6, such as at least 2, such as at least 2,5, such as at least 3, such as at least 3,3, such as at least 3,5 mg/ml

In one embodiment the liquid pharmaceutical formulation has a pH $\geq$ 7,4, such as pH $\geq$ 8,0, such as $\geq$ 8,3, and the Annexin A1 N-terminal-peptide has an aqueous solubility of at least 4,0 mg/ml, such as at least 4,5 mg/ml, such as at least 5,0 mg/ml, such as at least 6,0 mg/ml, such as at least 7,0 mg/ml, such as at least 8,0 mg/ml, such as at least 9,0 mg/ml, such as at least 10,0 mg/ml, such as at least 15,0 mg/ml; and/or said formulation having a concentration of said Annexin A1 N-terminal-peptide of at least 4,0 mg/ml, such as at least 4,5 mg/ml, such as at least 5,0 mg/ml, such as at least 6,0 mg/ml, such as at least 7,0 mg/ml, such as at least 8,0 mg/ml, such as at least 9,0 mg/ml, such as at least 10,0 mg/ml, such as at least 15,0 mg/ml.

*Solvent*

The liquid pharmaceutical formulation comprises a solvent. In one embodiment said solvent is water, such as ultrapure water.

Ultrapure water (UPW), high-purity water or highly purified water (HPW) is water that has been purified to stringent specifications, and has the highest levels of purity for all contaminant types, including: organic and inorganic compounds; dissolved and particulate matter; volatile and non-volatile, reactive and inert; hydrophilic and hydrophobic; and dissolved gases.

The liquid pharmaceutical formulation comprises a solvent, which solvent preferably is not saline. In one embodiment the liquid pharmaceutical formulation comprises a nonsaline solvent.

In a preferred embodiment the solvent comprise little or essentially no salts.

In one embodiment said solvent is **essentially non-ionic,** or low-ionic. In one embodiment said solvent has low-ionic strength. In one embodiment reference herein to 'essentially non-ionic' and 'low-ionic' is meant to refer to a solvent having low ionic strength.

The ionic strength of a solution is a measure of the concentration of ions in that solution. Ionic compounds, when dissolved in water, dissociate into ions. One of the main characteristics of a solution with dissolved ions is the ionic strength. Ionic strength can be molar (mol/L solution) or molal (mol/kg solvent). The molar ionic strength, *I*, of a solution is a function of the concentration of all ions present in that solution.

In one embodiment the solvent has an ionic strength of 1 $\mu$M to 50 mM.

In one embodiment the solvent has an ionic strength of 1 $\mu$M to 50 mM, such as 1 $\mu$M to 5 $\mu$M, such as 5 to 10 $\mu$M, such as 10 to 25 $\mu$M, such as 25 to 50 $\mu$M, such as 50 to 75 $\mu$M, such as 75 to 100 $\mu$M, such as 100 to 200 $\mu$M, such as 200 to 300 $\mu$M, such as 300 to 400 $\mu$M, such as 400 to 500 $\mu$M, such as 500 to 750 $\mu$M, such as 750 to 1000 $\mu$M (1 mM), such as 1 mM to 2 mM, such as 2 to 3 mM, such as 3 to 4 mM, such as 4 to 5 mM, such as 5 to 10 mM, such as 10 to 20 mM, such as 20 to 30 mM, such as 30 to 40 mM, such as 40 to 50 mM.

In one embodiment said solvent is an **essentially isotonic** solution. In one embodiment said solution has a tonicity of 100% +/- 100%. In one embodiment said solvent is a solution having a tonicity of 100% +/- 100%.

Isotonicity means having equal tension; denoting solutions possessing the same osmotic pressure or tonicity; more specifically, limited to solutions in which eukaryotic cells neither swell nor shrink (no net flow of water across the semipermeable cell membrane). Thus, a solution that is isosmotic with intracellular fluid will not be isotonic if it includes solute, such as urea, that freely permeates cell membranes.

An isotonic solution can be defined as having a tonicity of 100%, or approx. 300 mOsm.

In one embodiment said solution has a tonicity of approx. 100%, such as a tonicity of 100% +/- 100%, such as a tonicity of 0,1 % to 200 %; such as 80-85%, such as 85-90%, such as 90-95%, such as 95-100%, such as 100-105%, such as 105-110%, such as 110-115%, such as 115-120% tonicity.

In one embodiment said solution has a tonicity of 100% +/- 20%, such as a tonicity of 80% to 120%; such as 80-85%, such as 85-90%, such as 90-95%, such as 95-100%, such as 100-105%, such as 105-110%, such as 110-115%, such as 115-120% tonicity.

A tonicity of 100% +/- 20% is acceptable for regulatory purposes, while a tonicity of 100% +/- 100% is acceptable with justification.

In one embodiment said solution is 0-20% hypertonic, such as 0-5%, such as 5-10%, such as 10-15%, such as 15-20% hypertonic.

In one embodiment said solution is 0-20% hypotonic, such as 0-5%, such as 5-10%, such as 10-15%, such as 15-20% hypotonic.

In one embodiment said solvent comprises an **isotonicity modifier.** In one embodiment said solvent is a essentially isotonic solution comprising an isotonicity modifier.

In one embodiment said isotonicity modifier is selected from the group consisting of carbohydrates and sugar alcohols.

Sugar alcohols and carbohydrates share the same feature in their backbones, i. e.,-CHOH-CHOH-. The sugar alcohols include such compounds as sorbitol, mannitol, glycerol, and polyethylene glycols (PEGs). These compounds are straight-chain molecules. The carbohydrates, such as sucrose, mannose, ribose, trehalose, maltose, glycerol, inositol, glucose and lactose, on the other hand, are cyclic molecules that may contain a keto or aldehyde group. These two classes of compounds are effective in stabilizing protein against denaturation caused by elevated temperature and by freezethaw or freeze-drying processes.

In one embodiment said isotonicity modifier is selected from the group consisting of a carbohydrate, a sugar alcohol, and a combination of a carbohydrate and a sugar alcohol.

In one embodiment said solvent comprises a carbohydrate isotonicity modifier.

In one embodiment said solvent comprises a sugar alcohol isotonicity modifier.

In one embodiment said solvent comprises a carbohydrate and a sugar alcohol isotonicity modifier.

In one embodiment said sugar alcohol is not a liquid sugar alcohol, as these are not as useful for lyophilisation. In one embodiment said sugar alcohol is a solid sugar alcohol, such as a sugar alcohol which is solid at the conditions required for lyophilisation.

In one embodiment said isotonicity modifier is selected from the group consisting of pentose monosaccharides, hexose monosaccharides, disaccharides and trisaccharides.

In one embodiment said solution comprises one or more **pentose** monosaccharides (MW 150,13).

In one embodiment said solution comprises one or more pentose monosaccharides having a total weight percentage (w/v) of approx. 4.5% (isotonic).

In one embodiment said solution comprises one or more pentose monosaccharides having a total weight percentage (w/v) of 0,1 to 9%, such as 0,1 to 0,5, such as 0,5 to 1,0, such as 1,5 to 2,0, such as 1,5, to 2,0, such as 2,0 to 2,5, such as 2,5 to 3,0, such as 3,0 to 3,5, such as 3,5 to 4,0, such as 4,0 to 4,5, such as 4,5 to 5,0, such as 5,0 to 5,5, such as 5,5 to 6,0, such as 6,0 to 6,5, such as 7,0 to 7,5, such as 7,5 to 8,0, such as 8,0 to 8,5, such as 8,5 to 9,0% (w/v).

In one embodiment said solution comprises one or more pentose monosaccharides having a total weight percentage (w/v) of 3,6 to 5,4%; such as 3,6 to 3,8, such as 3,8 to 4,0, such as 4,0 to 4,2, such as 4,2 to 4,4, such as 4,4 to 4,6, such as 4,6 to 4,8, such as 4,8 to 5,0, such as 5,0 to 5,2% (w/v).

In one embodiment said solution comprises one or more **hexose** monosaccharides (MW 182,17).

In one embodiment said solution comprises one or more hexose monosaccharides having a total weight percentage (w/v) of approx. 5.5% (isotonic).

In one embodiment said solution comprises one or more hexose monosaccharides having a total weight percentage (w/v) of 0,1 to 10,9%, such as 0,1 to 0,5, such as 0,5 to 1,0, such as 1,5 to 2,0, such as 1,5, to 2,0, such as 2,0 to 2,5, such as 2,5 to 3,0, such as 3,0 to 3,5, such as 3,5 to 4,0, such as 4,0 to 4,5, such as 4,5 to 5,0, such as 5,0 to 5,5, such as 5,5 to 6,0, such as 6,0 to 6,5, such as 7,0 to 7,5, such as 7,5 to 8,0, such as 8,0 to 8,5, such as 8,5 to 9,0, such as 9,0 to 9,5, such as 9,5 to 10,0, such as 10,0 to 10,5, such as 10,5 to 10,9 % (w/v).

In one embodiment said solution comprises one or more hexose monosaccharides having a total weight percentage (w/v) of 4,4 to 6,6%, such as such as 4,4 to 4,6, such as 4,6 to 4,8, such as 4,8 to 5,0, such as 5,0 to 5,2, such as 5,2 to 5,4, such as 5,4 to 5,6, such as 5,6 to 5,8, such as 5,8 to 6,0, such as 6,0 to 6,2, such as 6,2 to 6,4, such as 6,4 to 6,6% (w/v).

In one embodiment said solution comprises one or more **disaccharides** (342,3).

In one embodiment said solution comprises one or more disaccharides having a total weight percentage (w/v) of approx. 10,3% (isotonic).

In one embodiment said solution comprises one or more disaccharides having a total weight percentage (w/v) of 0,1 to 20,5%, such as 0,1 to 1, such as 1-2, such as 2-3, such as 3-4, such as 4-5, such as 5-6, such as 6-7, such as 7-8, such as 8-9, such as 9-10, such as 10-11, such as 11-12, such as 12-13, such as 13-14, such as 14-15, such as 15-16, such as 16-17, such as 17-18, such as 18-19, such as 19-20, such as 20-20,5% (w/v).

In one embodiment said solution comprises one or more disaccharides having a total weight percentage (w/v) of 8,2 to 12,3%, such as 8,2 to 8,5, such as 8,5 to 9, such as 9 to 9,5, such as 9,5 to 10, such as 10 to 10,5, such as 10,5 to 11, such as 11 to 11,5, such as 11,5 to 12, such as 12 to 12,3% (w/v).

In one embodiment said solution comprises one or more **trisaccharides** (MW 504,42).

In one embodiment said solution comprises one or more trisaccharides having a total weight percentage (w/v) of approx. 15,1% (isotonic).

In one embodiment said solution comprises one or more trisaccharides having a total weight percentage (w/v) of 0,1 to 30,3%, such as such as 0,1 to 1, such as 1-2, such as 2-3, such as 3-4, such as 4-5, such as 5-6, such as 6-7, such as 7-8, such as 8-9, such as 9-10, such as 10-11, such as 11-12, such as 12-13, such as 13-14, such as 14-15, such as 15-16, such as 16-17, such as 17-18, such as 18-19, such as 19-20, such as 20-21, such as 21-22, such as 22-23, such as 23-24, such as 24-25, such as 25-26, such as 26-27, such as 27-28, such as 28-29, such as 29-30, such as 30-30,3% (w/v).

In one embodiment said solution comprises one or more trisaccharides having a total weight percentage (w/v) of 12,1 to 18,2%, such as 12,1 to 12,5, such as 12,5 to 13, such as 13 to 13,5, such as 13,5 to 14, such as 14 to 14,5, such as 14,5-15, such as 15 to 15,5, such as 15,5 to 16, such as 16 to 16,5, such as 16,5 to 17, such as 17,5 to 18, such as 18 to 18,2% (w/v).

In one embodiment said carbohydrate isotonicity modifier is selected from the group consisting of: sucrose, trehalose, mannose, ribose, maltose, glucose, lactose, galactose and arabinose.

In one embodiment said sugar alcohol isotonicity modifier is selected from the group consisting of: mannitol, sorbitol, inositol, glycerol, xylitol, propylene glycol, polyethylene glycols (PEGs) and polypropylene/ethylene glycol copolymer.

In a particular embodiment said solution comprises sucrose or trehalose.

In a particular embodiment said solution comprises sucrose. Sucrose is shown herein to be efficient in providing chemical stability.

In one embodiment the solution comprises approx. 10% (w/v) sucrose.

In one embodiment the solution comprises 8-12% (w/v) sucrose, such as 8-9, such as 9-10, such as 10-11, such as 11-12% (w/v) sucrose.

In one embodiment the solution comprises approx. 1% (w/v) sucrose.

In one embodiment the solution comprises 1-5% (w/v) sucrose, such as 1-2, such as 2-3, such as 3-4, such as 4-5 (w/v) sucrose.

In one embodiment the solution comprises 0-20% (w/v) sucrose, such as 0,1-1, 1-2, 2-3, 3-4, 4-5, 5-6, 6-7, 7-8, 8-9, 8-9, 9-10, 10-11, 11-12, 12-13, 13-14, 14-15, 15-16, 16-17, 17-18, 18-19, 19-20% (w/v) sucrose.

In a particular embodiment said solution comprises mannitol.

In one embodiment the solvent comprises approx. 5% (w/v) mannitol.

In one embodiment the solution comprises 4-6% (w/v) mannitol, such as 4 to 6% (w/v) mannitol, such as 4 to 4,5, such as 4,5 to 5, such as 5 to 5,5, such as 5,5 to 6% mannitol.

In one embodiment the solvent comprises approx. 4% (w/v) mannitol.

In one embodiment the solution comprises 3-5% (w/v) mannitol, such as 3 to 5% (w/v) mannitol, such as 3 to 3,5, such as 3,5 to 4, such as 4 to 4,5, such as 4,5 to 4% mannitol.

In one embodiment the solution comprises 0-10% (w/v) mannitol, such as 0,1-1, such as 1-2, such as 2-3, such as 3-4, such as 4-5, such as 5-6, such as 6-7, such as 7-8, such as 8-9, such as 9-10% (w/v) mannitol.

In one embodiment said solution comprises mannitol, and sucrose or trehalose.

In one embodiment said solution comprises mannitol and sucrose.

In one embodiment the solution comprises 0-20% (w/v) sucrose, such as 0,1-1, 1-2, 2-3, 3-4, 4-5, 5-6, 6-7, 7-8, 8-9, 8-9, 9-10, 10-11, 11-12, 12-13, 13-14, 14-15, 15-16, 16-17, 17-18, 18-19, 19-20% (w/v) sucrose, and comprises 0-10% (w/v) mannitol, such as 0,1-1, such as 1-2, such as 2-3, such as 3-4, such as 4-5, such as 5-6, such as 6-7, such as 7-8, such as 8-9, such as 9-10% (w/v) mannitol.

In one embodiment said solution comprises approx. 4% (w/v) mannitol and approx. 2,5% sucrose.

In one embodiment said solution comprises 3 to 5% (w/v) mannitol and 2 to 3% (w/v) sucrose.

In one embodiment said solution comprises approx. 4% (w/v) mannitol and approx. 1% sucrose.

In one embodiment said solution comprises 3 to 5 % (w/v) mannitol and 0,5 to 2% sucrose.

In one embodiment said solution comprises 0 to 8 % (w/v) mannitol and 0 to 5% (w/v) sucrose. In one embodiment said solution comprises 0 to 8 % (w/v) mannitol, such as 0 to 1, such as 1 to 2, such as 2 to 3, such as 3 to 4, such as 4 to 5, such as 5 to 6, such as 6 to 7, such as 7 to 8% (w/v) mannitol; and 0 to 5% (w/v) sucrose, such as 0 to 1, such as 1 to 2, such as 2 to 3, such as 3 to 4, such as 4 to 5 (w/v) sucrose.

In a particular embodiment said isotonicity modifier preserves the peptide against chemical degradation during lyophilisation. It is observed that carbohydrates are wellsuited for this purpose. In one embodiment the liquid pharmaceutical formulation comprises one or more carbohydrates, such as one or more carbohydrates that improve chemical stability, such as that preserve the peptide during lyophilisation.

In one embodiment the liquid pharmaceutical formulation comprises one or more carbohydrates selected from the group consisting of sucrose and trehalose. In one embodiment the liquid pharmaceutical formulation comprises sucrose.

In one embodiment the liquid pharmaceutical formulation comprises one or more buffers, such as a buffer.

The choice of buffer can also affect stability of peptide formulations in part because the choice of buffer affects the pH. The buffers used in the present disclosure preferably provides a buffering capacity above 6, for example above 7, such as above 8. There are few pharmaceutically acceptable buffers in the alkaline range, and phosphate buffers typically used in peptide formulations cannot maintain a pH above 8.

In one embodiment said buffer aids in stabilizing the pH of the liquid formulation. pH is important for the physical stability of the AnxA1 N-terminal-peptide.

In one embodiment the liquid pharmaceutical formulation comprises one or more buffers selected from the group consisting of Acetate buffer, TRIS, Amine-containing buffers, Amino-acid based buffers such as lysine, hydroxy-lysine histidine and glycylglycine, non-phosphate buffers, bicarbonate buffers, Polysorbate-80 (e.g. Tween 80), HEPES and borate.

In one embodiment the liquid pharmaceutical formulation comprises glycyl-glycine.

In one embodiment the liquid pharmaceutical formulation comprises Tween20.

In one embodiment the liquid pharmaceutical formulation comprises glycyl-glycine and Tween20. In one embodiment the liquid pharmaceutical formulation comprises 1 to 20 mM glycyl-glycine and 0,001 to 0,1% Tween20. In one embodiment the liquid pharmaceutical formulation comprises 10mM glycyl-glycine and 0,01% Tween20.

In one embodiment the liquid pharmaceutical formulation comprises histidine.

In one embodiment the liquid pharmaceutical formulation further comprises a preservative. In one embodiment said preservative is selected from the group consisting of m-cresol, benzyl alcohol, methyl, ethyl, propyl parabens, butyl parabens, methyl parabens, and phenol.

*Preparing liquid formulation*

It is also an aspect of the present disclosure to provide a process of preparing a liquid pharmaceutical formulation as defined herein above, said process comprising the steps of

a. mixing an Annexin A1 N-terminal-peptide, as defined herein above, with a solvent selected from the group consisting of

i. water, such as ultrapure water; and
ii. a solution having an ionic strength of 1 $\mu$M to 50 mM, wherein the solvent comprises an isotonicity modifier selected from a carbohydrate and/or a sugar alcohol,

b. optionally filtering the mixture of step a., and
c. adjusting the pH of said liquid pharmaceutical formulation to pH < 6.

It is also an aspect of the present disclosure to provide a process of preparing a liquid pharmaceutical formulation as defined herein above, said process comprising the steps of

a. mixing an Annexin A1 N-terminal-peptide, as defined herein above, with a solvent, wherein said solvent is a solution having an ionic strength of 1 $\mu$M to 50 mM
b. optionally filtering the mixture of step a., and
c. adjusting the pH of said liquid pharmaceutical formulation to pH $\geq$ 8.

In one embodiment the pH of said liquid pharmaceutical formulation is adjusted to 6 > pH $\leq$ 6,5, such as 6,5 $\geq$ pH $\leq$ 7, such as 7 $\geq$ pH $\leq$ 7,5, such as 7,5 $\geq$ pH $\leq$ 8, such as 8 $\geq$ pH $\leq$ 8,5, such as 8,5 $\geq$ pH $\leq$ 9, such as 9 $\geq$ pH $\leq$ 9,5, such as 9,5 $\geq$ pH $\leq$ 10, such as 10 $\geq$ pH $\leq$ 10,5, such as 10,5 $\geq$ pH $\leq$ 11, such as 11 $\geq$ pH $\leq$ 11,5, such as 11,5 $\geq$ pH $\leq$ 12.

In one embodiment the pH of said liquid pharmaceutical formulation is adjusted to $\geq$ 7,4. In one embodiment the pH of said liquid pharmaceutical formulation is adjusted to $\geq$ 7,5, such as $\geq$ 8, such as $\geq$ 8,5, such as $\geq$ 9.

In one embodiment the process comprises mixing of said Annexin A1 N-terminal-peptide at a concentration of at least 0,1 mg/ml, such as at least 0,5 mg/ml, such as at least 1,0 mg/ml, such as at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml.

In one embodiment the process comprises mixing of said Annexin A1 N-terminal-peptide at a concentration of 0,1 to 50 mg/ml; such as in a concentration of 0,1 to 0,5 mg/ml, such as 0,5 to 1 mg/ml, such as 1 to 2 mg/ml, such as 2 to 3, such as 3 to 4, such as 4 to 5, such as 5 to 6, such as 6 to 7, such as 7 to 8, such as 8 to 9, such as 9 to 10, such as 10 to 11, such as 11 to 12, such as 12 to 13, such as 13 to 14, such as 14 to 15, such as 15 to 16, such as 16 to 17, such as 17 to 18, such as 18 to 19, such as 19 to 20 mg/ml, such as 20 to 21, such as 21 to 22, such as 22 to 23, such as 23 to 24, such as 24 to 25, such as 25 to 26, such as 26 to 27, such as 27 to 28, such as 28 to 29, such as 29 to 30 mg/ml, such as 30 to 31, such as 31 to 32, such as 32 to 33, such as 33 to 34, such as 34 to 35, such as 35 to 36, such as 36 to 37, such as 37 to 38, such as 38 to 39, such as 39 to 40 mg/ml, such as 40 to 41, such as 41 to 42, such as 42 to 43, such as 43 to 44, such as 44 to 45, such as 45 to 46, such as 46 to 47, such as 47 to 48, such as 48 to 49, such as 49 to 50 mg/ml.

*Lyophilized formulation*

In one embodiment the liquid pharmaceutical formulation according to the present disclosure is lyophilised, or freeze-dried. Lyophilization is a water removal process typically used to preserve materials, to extend shelf life and/or make the material more convenient for transport. Lyophilization works by rapid freezing and dehydration of the frozen product under high vacuum (low pressure).

In one embodiment the liquid pharmaceutical formulation according to the present disclosure is lyophilised.

It is an aspect of the present disclosure to provide a process of preparing a lyophilized pharmaceutical formulation, said method comprising the steps of

  a. providing a liquid pharmaceutical formulation of pH > 6, said formulation comprising a therapeutically effective amount of an Annexin A1 N-terminal-peptide selected from the group consisting of

AMVSEFLKQAWFIENEEQEYVQTVKSSKGGPGSAVSPYPTFNPSSDV AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV AA (SEQ ID NO:7; Annexin A1 2-50 V24L),

AMVSEFLKQAWFIENEEQEYVQTLKSSKGGPGSAVSPYPTFNPSSDV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and a solvent selected from the group consisting of

    i. water, such as ultrapure water; and
    ii. a solution having an ionic strength of 1 $\mu$M to 50 mM,

  wherein said solvent comprises one or more isotonicity modifiers selected from a carbohydrate and/or a sugar alcohol,
  and optionally a buffer, and

  b. lyophilizing said liquid pharmaceutical formulation of step a.

It is an aspect of the present disclosure to provide a process of preparing a lyophilized pharmaceutical formulation, said method comprising the steps of

  a. providing a liquid pharmaceutical formulation of pH $\geq$ 8, said formulation comprising a therapeutically effective amount of an Annexin A1 N-terminal-peptide selected from the group consisting of

AMVSEFLKQAWFIENEEQEYVQTVKSSKGGPGSAVSPYPTFNPSSDV AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV

AA (SEQ ID NO:7; Annexin A1 2-50 V24L),

AMVSEFLKQAWFIENEEQEYVQTLKSSKGGPGSAVSPYPTFNPSSDV (SEQ ID NO:8; Annexin A1 2-48 V24L), and

AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and solvent, wherein said solvent is a solution having an ionic strength of 1 $\mu$M to 50 mM,
and optionally a buffer, and

b. lyophilizing said liquid pharmaceutical formulation of step a.

In one embodiment there is provided a process of preparing a lyophilized pharmaceutical formulation, said method comprising the steps of

a. freezing a liquid pharmaceutical formulation as defined herein above,
b. primary drying (sublimation phase), comprising lowering pressure and heating, and
c. secondary drying (desorption phase).

*Reconstituted liquid formulation / solution*

It is a further aspect of the present disclosure to provide a method of making a reconstituted solution comprising an Annexin A1 N-terminal-peptide, said method comprising

a. providing a lyophilized pharmaceutical formulation according to the present disclosure, and
b. dissolving said lyophilized pharmaceutical formulation in a solvent.

In one embodiment the resulting solution has a pH > 6. In one embodiment said solution has a pH of $6 > pH \leq 6,5$, such as $6,5 \geq pH \leq 7$, such as $7 \geq pH \leq 7,5$, such as $7,5 \geq pH \leq 8$, such as $8 \geq pH \leq 8,5$, such as $8,5 \geq pH \leq 9$, such as $9 \geq pH \leq 9,5$, such as $9,5 \geq pH \leq 10$, such as $10 \geq pH \leq 10,5$, such as $10,5 \geq pH \leq 11$, such as $11 \geq pH \leq 11,5$, such as $11,5 \geq pH \leq 12$.

In one embodiment said solvent is selected from the group consisting of

a. water, such as ultrapure water; and
b. a solution having an ionic strength of 1 $\mu$M to 50 mM,
wherein said solvent comprises one or more isotonicity modifiers selected from a carbohydrate and/or a sugar alcohol I.

In a particular embodiment said solvent is water, such as ultrapure water.

In one embodiment said solvent is a solution having an ionic strength of 1 $\mu$M to 50 mM.

In one embodiment the solvent has a pH > 6. In one embodiment said solvent has a pH of $6 > pH \leq 6,5$, such as $6,5 \geq pH \leq 7$, such as $7 \geq pH \leq 7,5$, such as $7,5 \geq pH \leq 8$, such as $8 \geq pH \leq 8,5$, such as $8,5 \geq pH \leq 9$, such as $9 \geq pH \leq 9,5$, such as $9,5 \geq pH \leq 10$, such as $10 \geq pH \leq 10,5$, such as $10,5 \geq pH \leq 11$, such as $11 \geq pH \leq 11,5$, such as $11,5 \geq pH \leq 12$.

*Annexin A1 N-terminal peptide*

On the cell surface the Annexin A1 (AnxA1) protein is exposed to extracellular fluids and in the presence of calcium undergoes structural re-organization, consequent to interaction with phospholipids via the core region of the protein (~280 amino acid long), which leads to the exposure of the N-terminal region (~50 amino acid long). This conformational change is thought to lead to the interaction of the AnxA1 N-terminus with specific receptors. The N-terminus of AnxA1 comprises amino acids 1-54 (SEQ ID NO:2).

In addition to representing the pharmacophore of the protein affording interaction with counter-ligands, the N-terminus is also a highly regulated region. It can undergo phosphorylation on specific Tyrosine or Serine sites, a pre-requisite for secretion in certain cell types, or can be cleaved by serine proteases. A number of proteases have been shown to cleave at specific sites within the AnxA1 N-terminal region and it is plausible that this process is not an *in vitro* artifact but of biological significance.

Both human recombinant AnxA1 and the N-terminal peptide Ac2-26 exert anti-inflammatory and pro-resolving effects in a variety of experimental models.

A number of peptides primarily derived from the unique N-terminal portion of the Annexin A1 protein have been shown to possess anti-inflammatory properties.

An *Annexin A1 N-terminal-peptide* as defined herein is a peptide comprising or consisting of the amino acid sequence AMVSEFLKQ AWFIENEEQE YVQTVKSSKG GPGSAVSPYP TFNPSSDVAA LHKA (AnxA1 2-54; SEQ ID NO:3), including any shorter fragments thereof, any variants thereof, as well as any variant of said shorter fragment thereof.

In one embodiment said Annexin A1 N-terminal-peptide is selected from the group consisting of:

AMVSEFLKQAWFIENEEQEYVQTVKSSKGGPGSAVSPYPTFNPSSDVAA (SEQ ID NO:4; Annexin A1 2-50),
AMVSEFLKQAWFIENEEQEYVQTVKSSKGGPGSAVSPYPTFNPSSDV (SEQ ID NO:5; Annexin A1 2-48), and
AMVSEFLKQAWFIENEEQEYVQTVKSSKGGPGSAVSPYPTFNPSS (SEQ ID NO:6; Annexin A1 2-46),
and a variant of any one of SEQ ID NOs:4-6, wherein said variant comprises 1 to 6 individual amino acid substitutions, such as 1 amino acid substitution, such as 2 amino acid substitutions, such as 3 amino acid substitutions, such as 4 amino acid substitutions, such as 5 amino acid substitutions, such as 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3).

In one embodiment said variant comprises 1 to 6 individual amino acid substitutions, such as 1 to 2 amino acid substitutions, such as 2 to 3 amino acid substitutions, such as 3 to 4 amino acid substitutions, such as 4 to 5 amino acid substitutions, such as 5 to 6 individual amino acid substitutions.

In one embodiment said one or more amino acid substitutions are conservative amino acid substitutions. In one embodiment said one or more amino acid substitutions are non-conservative amino acid substitutions.

A variant of a peptide as defined herewith can in principle have one or more substitutions at one or more positions. Individual amino acid residues in the disclosed sequences can be substituted with any given proteinogenic or non-proteinogenic amino acid.

The genetic code specifies 20 standard amino acids naturally incorporated into polypeptides (proteinogenic): Ala, Arg, Asn, Asp, Cys, Gln, Glu, Gly, His, Ile, Leu, Lys, Met, Phe, Pro, Ser, Tyr, Thr, Trp, Val, and 2 which are incorporated into proteins by unique synthetic mechanisms: Sec (selenocysteine, or U) and Pyl (pyrrolysine, O). These are all L-stereoisomers.

Aside from the 22 standard or natural amino acids, there are many other non-naturally occurring amino acids (non-proteinogenic or non-standard). They are either not found in proteins, or are not produced directly and in isolation by standard cellular machinery. Non-standard amino acids are usually formed through modifications to standard amino acids, such as post-translational modifications. Examples of unnatural amino acid residues are Abu, Aib, Nle (Norleucine), DOrn (D-ornithine, deguanylated arginine), Nal (beta-2-naphthyl-alanine), D-Nal (beta-2-naphthyl-D-alanine), DArg, DTrp, DPhe and DVal.

Any amino acids according to the present disclosure may be in the L- or D-configuration. If nothing is specified, reference to the L-isomeric form is preferably meant.

The term polypeptide also embraces post-translational modifications introduced by chemical or enzyme-catalyzed reactions, as are known in the art. Such post-translational modifications can be introduced prior to partitioning, if desired. Also, functional equivalents may comprise chemical modifications such as ubiquitination, labeling (e.g., with radionuclides, various enzymes, etc.), pegylation (derivatization with polyethylene glycol), or by insertion (or substitution by chemical synthesis) of amino acids, which do not normally occur in human proteins (e.g. ornithine).

Polypeptides as defined herein with N-terminal alkylations and C-terminal esterifications are also encompassed within the present disclosure. Functional equivalents also comprise glycosylated and covalent or aggregative conjugates formed with the same molecules, including dimers or unrelated chemical moieties. Such functional equivalents are prepared by linkage of functionalities to groups which are found in a fragment including at any one or both of the N- and C-termini, by means known in the art.

In one embodiment said Annexin A1 N-terminal-peptide comprises at least 1 amino acid substitution, wherein the valine (V) at position 24 of any one of SEQ ID NOs:3-6 and 10 is substituted with any other standard or non-standard amino acid.

In one embodiment said Annexin A1 N-terminal-peptide comprises at least 1 amino acid substitution, wherein the valine (V) at position 24 of any one of SEQ ID NOs:3-6 and 10 is substituted with an amino acid residue independently selected from the group consisting of glycine, alanine, serine, threonine, cysteine, leucine, isoleucine, methionine, proline, phenylalanine, tyrosine, tryptophan, aspartic acid, glutamic acid, asparagine, glutamine, histidine, lysine and arginine.

In one embodiment said Annexin A1 N-terminal-peptide comprises at least 1 amino acid substitution, wherein the valine (V) at position 24 of any one of SEQ ID NOs:3-6 and 10 is substituted with leucine (L) (Val24Leu or V24L). In some embodiments the Val24Leu mutation increases protease stability.

In one embodiment said Annexin A1 N-terminal-peptide is selected from the group consisting of:

AMVSEFLKQAWFIENEEQEYVQTLKSSKGGPGSAVSPYPTFNPSSDVAA (SEQ ID NO:7; Annexin A1 2-50 V24L),
AMVSEFLKQAWFIENEEQEYVQT**L**KSSKGGPGSAVSPYPTFNPSSDV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L),
and a variant of any one of SEQ ID NOs:7-9, wherein said variant comprises 1 to 6 individual amino acid substitutions, such as 1 amino acid substitution, such as 2 amino acid substitutions, such as 3 amino acid substitutions, such as 4 amino acid substitutions, such as 5 amino acid substitutions, such as 6 individual amino acid substitutions; such as wherein said variant comprises V24L,
wherein said variant is a ligand and/or agonist of one or more of Formyl Peptide Receptor 1 (FPR1), Formyl Peptide Receptor 2 (FPR2) and Formyl Peptide Receptor 3 (FPR3).

In one embodiment said one or more amino acid substitutions are conservative amino acid substitutions.

In one embodiment the alanine residue at position 10 of any one of SEQ ID NOs:4-9 is substituted with any other standard or non-standard amino acid.

In one embodiment the alanine residue at position 10 of any one of SEQ ID NOs:4-9 is substituted with an amino acid residue independently selected from the group consisting of leucine, aspartic acid, methionine, glutamic acid, isoleucine and arginine.

In one embodiment the amino acid residue at position 10 of any one of SEQ ID NOs:4-9 is not alanine; i.e. is any amino acid except for alanine.

In one embodiment the valine residue at position 21 of any one of SEQ ID NOs:4-9 is substituted with any other standard or non-standard amino acid.

In one embodiment the valine residue at position 21 of any one of SEQ ID NOs:4-9 is substituted with an amino acid residue independently selected from the group consisting of leucine, aspartic acid, methionine, glutamic acid, isoleucine and lysine.

In one embodiment the residue at position 21 of any one of SEQ ID NOs:4-9 is not valine, i.e. is any amino acid except for valine.

In one embodiment the alanine residue at position 10 and the valine residue at position 21 of any one of SEQ ID NOs:4-9 are both substituted with an amino acid residue independently selected from the group consisting of leucine, aspartic acid, methionine, glutamic acid, isoleucine, arginine and lysine; such as the group consisting of leucine, aspartic acid and methionine; such as leucine. In one embodiment residue 10 and residue 21 of any one of SEQ ID NOs:4-9 are identical.

In one embodiment the valine residue at position 35 of any one of SEQ ID NOs:4-9 is substituted with any other standard or non-standard amino acid.

In one embodiment the valine residue at position 35 of any one of SEQ ID NOs:4-9 is substituted with an amino acid residue independently selected from the group consisting of glycine, alanine, serine, threonine, cysteine, leucine, isoleucine, methionine, proline, phenylalanine, tyrosine, tryptophan, aspartic acid, glutamic acid, asparagine, glutamine, histidine, lysine and arginine.

In one embodiment the residue at position 35 of any one of SEQ ID NOs:4-9 is lysine.

In one embodiment the residue at position 35 of any one of SEQ ID NOs:4-9 is not valine, i.e. is any amino acid except for valine.

In one embodiment said Annexin A1 N-terminal-peptide is selected from the group consisting of:

AMVSEFLKQAWFIENEEQEYVQTVKSSKGGPGSAVSPYPTFNPSSDVAA (SEQ ID NO:4; Annexin A1 2-50),
AMVSEFLKQAWFIENEEQEYVQTVKSSKGGPGSAVSPYPTFNPSSDV (SEQ ID NO:5; Annexin A1 2-48),
AMVSEFLKQAWFIENEEQEYVQTLKSSKGGPGSAVSPYPTFNPSSOVAA (SEQ ID NO:7; Annexin A1 2-50 V24L), and
AMVSEFLKQAWFIENEEQEYVQT**L**KSSKGGPGSAVSPYPTFNPSSDV (SEQ ID NO:8; Annexin A1 2-48 V24L),
and a variant of any one of SEQ ID NOs:7-9, wherein said variant comprises 1 to 6 individual amino acid substitutions, such as 1 amino acid substitution, such as 2 amino acid substitutions, such as 3 amino acid substitutions, such as 4 amino acid substitutions, such as 5 amino acid substitutions, such as 6 individual amino acid substitutions; such as wherein said variant comprises V24L,
wherein said variant is a ligand and/or agonist of one or more of Formyl Peptide Receptor 1 (FPR1), Formyl Peptide Receptor 2 (FPR2) and Formyl Peptide Receptor 3 (FPR3).

In one embodiment said Annexin A1 N-terminal-peptide is selected from the group consisting of:

AMVSEFLKQAWFIENEEQEYVQTVKSSKGGPGSAVSPYPTFNPSSDVAA (SEQ ID NO:4; Annexin A1 2-50) and
AMVSEFLKQAWFIENEEQEYVQTVKSSKGGPGSAVSPYPTFNPSSDV (SEQ ID NO:5; Annexin A1 2-48).

In one embodiment said Annexin A1 N-terminal-peptide is selected from the group consisting of:

AMVSEFLKQAWFIENEEQEYVQTLKSSKGGPGSAVSPYPTFNPSSOVAA (SEQ ID NO:7; Annexin A1 2-50 V24L), and
AMVSEFLKQAWFIENEEQEYVQT**L**KSSKGGPGSAVSPYPTFNPSSDV (SEQ ID NO:8; Annexin A1 2-48 V24L).

In one embodiment said Annexin A1 N-terminal-peptide is AMVSEFLKQAWFIENEEQEYVQT**L**KSSKGGPGSAVSPYPTFNPSSDV (SEQ ID NO:8; Annexin A1 2-48 V24L).

A fragment and a variant of an Annexin A1 N-terminal-peptide as disclosed herein are in one embodiment equivalent to a functional fragment and/or a functional variant of said Annexin A1 N-terminal-peptide.

A functional variant or functional fragment means that the variant or fragments essentially retains the functionality of the peptide from which it is derived.

An Annexin A1 N-terminal-peptide, or a variant or a fragment thereof as defined herewith, including functional variants thereof, according to the present disclosure in one embodiment activates and/or stimulates one or more of Formyl Peptide Receptor 1 (FPR1), Formyl Peptide Receptor 2 (FPR2) and Formyl Peptide Receptor 3 (FPR3).

An Annexin A1 N-terminal-peptide, or a variant or a fragment thereof as defined herewith, is in one embodiment a ligand and/or agonist of one or more of Formyl Peptide Receptor 1 (FPR1), Formyl Peptide Receptor 2 (FPR2) and Formyl Peptide Receptor 3 (FPR3).

The term "agonist" in the present context refers to a peptide as defined herein, capable of binding to, or in some embodiments, capable of binding to at least some extent and/or activating a receptor, or in some embodiments, activating a receptor to at least some extent. For example, a FPR2 agonist is thus capable of binding to and/or activating the FPR2.

An agonist may be an agonist of several different types of receptors, and thus capable of binding and/or activating several different types of receptors. Said agonist can also be a selective agonist which only binds and activates one type of receptor. The term "antagonist" in the present context refers to a substance capable of inhibiting the effect of a receptor agonist.

Full agonists bind (have affinity for) and activate a receptor, displaying full efficacy at that receptor. "Partial agonists" in the present context are peptides able to bind and activate a given receptor, but having only partial efficacy at the receptor relative to a full agonist. Partial agonists can act as antagonists when competing with a full agonist for receptor occupancy and producing a net decrease in the receptor activation compared to the effects or activation observed with the full agonist alone.

"Selective agonists" in the present context are compounds which are selective and therefore predominantly bind and activates one type of receptor. Thus a selective FPR2 agonist is selective for the FPR2.

Polypeptides according to the present disclosure are in one embodiment capable of binding and activating to some extent one or several formyl peptide receptors and can have different binding affinities and/or different receptor activation efficacy for different receptors. Affinity refers to the number and size of intermolecular forces between a peptide ligand and its receptor, and residence time of the ligand at its receptor binding site; and receptor activation efficacy refers to the ability of the peptide ligand to produce a biological response upon binding to the target receptor and the quantitative magnitude of this response. In some embodiments, such differences in affinity and receptor activation efficacy are determined by receptor binding/activation studies which are conventional in the art, for instance by generating $EC_{50}$ and Emax values for stimulation of ligand binding in cells expressing one or several types of receptors as mentioned herein, or on tissues expressing the different types of receptors. High affinity means that a lower concentration of a ligand is needed to obtain a binding of 50% of the receptors compared to ligand peptides which have lower affinity; high receptor activation efficacy means that a lower concentration of the peptide is needed to obtain a 50% receptor activation response (low $EC_{50}$ value), compared to peptides which have lower affinity and/or receptor activity efficacy (higher $EC_{50}$ value).

In one embodiment, the peptides can have differing affinities and/or receptor activation efficacies for two or more of the receptors selected from FPR1, FPR2 and FPR3.

The receptor activation potency of peptide agonists can also be measured in $p(A_{50})$ values which is a conventional method for determining the receptor activation efficacy of an agonist.

In a particular embodiment, a peptide according to the present disclosure has binding affinity and/or receptor efficacy for the Formyl Peptide Receptor 2 (FPR2).

In one particular embodiment, a peptide according to the present disclosure is capable of binding to and activating FPR2. In a further embodiment said peptide is a full agonist of FPR2.

In one embodiment an Annexin A1 N-terminal-peptide selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV

AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L),
and
AMVSEFLKQAWFIENEEQEYVQTLKSSKGGPGSAVSPYPTFNPSS (SEQ ID NO:9; Annexin A1 2-46 V24L),
and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3),

a. binds to one or more of the formyl peptide receptors, including FPR1, FPR2 and FPR3,
b. activates and/or stimulates one or more of the formyl peptide receptors, including FPR1, FPR2 and FPR3,
c. binds and/or activates FPR2,
d. activates immune cells
e. activates leukocytes, such as phagocytic leukocytes,
f. activates neutrophils and/or monocytes
g. activates phagocytic leukocytes' effector functions, such as inducing neutrophil chemotaxis, mobilization of neutrophil complement receptor 3 (CR3), and activation of the neutrophil NADPH-oxidase, and/or
h. induces chemotaxis in phagocytic leukocytes.

In one embodiment the Annexin A1 N-terminal-peptide of the present disclosure is N-terminally acetylated ($COCH_3$ or Ac-).
In one embodiment the Annexin A1 N-terminal-peptide of the present disclosure is C-terminally amidated ($-NH_2$).
It is as aspect of the present disclosure to provide a liquid pharmaceutical formulation of pH > 6,0; said formulation comprising

a. an Annexin A1 (AnxA1) N-terminal-peptide, such as a therapeutically effective amount of an Annexin A1 (AnxA1) N-terminal-peptide, selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV

AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L),
and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent selected from the group consisting of

i. water, such as ultrapure water; and

ii. a solution having an ionic strength of 1 μM to 50 mM,

wherein said solvent comprises one or more isotonicity modifiers selected from a carbohydrate and/or a sugar alcohol.

It is another aspect of the present disclosure to provide a liquid pharmaceutical formulation of pH ≥ 8; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48), AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent, wherein said solvent is a solution having an ionic strength of 1 μM to 50 mM.

Also disclosed is a liquid pharmaceutical formulation of pH > 6,0; said formulation comprising

a. an Annexin A1 (AnxA1) N-terminal-peptide, such as a therapeutically effective amount of an Annexin A1 (AnxA1) N-terminal-peptide, selected from the group consisting of

AMVSEFLKQAWFIENEEQEYVQTVKSSKGGPGSAVSPYPTFNPSSDV AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48), AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions; and

b. a solvent selected from the group consisting of

i. water, such as ultrapure water; and
ii. a solution having an ionic strength of 1 μM to 50 mM,

wherein said solvent comprises one or more isotonicity modifiers selected from a carbohydrate and/or a sugar alcohol optionally wherein the solution is water, said solution having a tonicity of 100% (isotonic) +/- 100%, and

optionally wherein the solution having an ionic strength of 1 μM to 50 mM has a tonicity of 100% (isotonic) +/- 100%.

In one embodiment said liquid pharmaceutical formulation has a pH of pH ≥ 7,4, such as pH ≥ 7,5, such as pH ≥ 7,6, such as pH ≥ 7,7, such as pH ≥ 7,8, such as pH ≥ 7,9, such as pH ≥ 8,0.

In one embodiment said liquid pharmaceutical formulation has a pH of pH ≥ 8.

In one embodiment said liquid pharmaceutical formulation has a pH of 8 to 8,5.

In one embodiment said liquid pharmaceutical formulation has a pH of approx. 8,3.

In one embodiment said liquid pharmaceutical formulation comprises essentially no fibrils and/or comprises essentially no proteinaceous particles of ≥ 1 μm, such as ≥ 10 μm, such as ≥ 100 μm.

In one embodiment said Annexin A1 N-terminal-peptide has an aqueous solubility of at least 0,1 mg/ml, such as at least 0,5 mg/ml, such as at least 1,0 mg/ml, such as at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml liquid pharmaceutical formulation.

In one embodiment said liquid pharmaceutical formulation has a tonicity of 100% +/-20%, such as a tonicity of 80% to 120%; such as 80-85%, such as 85-90%, such as 90-95%, such as 95-100%, such as 100-105%, such as 105-110%, such as 110-115%, such as a tonicity of 115-120%.

In one embodiment there is provided a liquid pharmaceutical formulation wherein

a. the solution has a pH of pH ≥ 7,4, such as pH ≥ 7,5, such as pH ≥ 7,6, such as pH ≥ 7,7, such as pH ≥ 7,8, such as pH ≥ 7,9, such as pH ≥ 8,0, such as pH ≥ 8,3, and/or

b. the solution has an ionic strength of 1 μM to 50 mM, and/or

c. the solution has a tonicity of 100% (isotonic) +/- 20%, and/or

d. the Annexin A1 N-terminal-peptide has an aqueous solubility of at least 0,1 mg/ml, such as at least 0,5 mg/ml, such as at least 1,0 mg/ml, such as at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml; and/or

e. the formulation has a concentration of said Annexin A1 N-terminal-peptide of 0,1 to 10 mg/ml, such as 0,1 to 0,5 mg/ml, such as 0,5 to 1 mg/ml, such as 1 to 2 mg/ml, such as 2 to 3 mg/ml, such as 3 to 4 mg/ml, such as 4 to 5 mg/ml, such as 5 to 6 mg/ml, such as 6 to 7 mg/ml, such as 7 to 8 mg/ml, such as 8 to 9 mg/ml, such as 9 to 10 mg/ml.

In one embodiment said liquid pharmaceutical formulation comprises one or more isotonicity modifiers.

In one embodiment said liquid pharmaceutical formulation comprises one or more isotonicity modifiers selected from the group consisting of: sucrose, trehalose, mannose, ribose, maltose, glucose, lactose, galactose, arabinose, mannitol, sorbitol, inositol, glycerol, xylitol, propylene glycol, polyethylene glycols (PEGs) and polypropylene/ ethylene glycol copolymer; such as comprises sucrose and/or mannitol.

In one embodiment said liquid pharmaceutical formulation comprises one or more isotonicity modifiers selected from the group consisting of: sucrose and mannitol.

In one embodiment said liquid pharmaceutical formulation comprises one or more buffers.

In one embodiment said liquid pharmaceutical formulation comprises one or more buffers selected from the group consisting of an acetate buffer, TRIS, amine-containing buffers, amino-acid based buffers such as lysine, hydroxy-lysine, histidine and glycylglycine, non-phosphate buffers, bicarbonate buffers, Polysorbate-80 (e.g. Tween 80), HEPES and borate.

In one embodiment said liquid pharmaceutical formulation comprises approx. 4% mannitol, approx. 1% sucrose, approx. 10 mM glycylglycine, approx. 0,01% polysorbate80, at a pH of approx 8,3.

In one embodiment said variant of any one of SEQ ID NOs:4-9 comprises 3 individual amino acid substitutions.

In one embodiment said variant of any one of SEQ ID NOs:4-9 comprises 2 individual amino acid substitutions.

In one embodiment said variant of any one of SEQ ID NOs:4-9 comprises 1 amino acid substitution.

In one embodiment said Annexin A1 (AnxA1) N-terminal-peptide is C-terminally amidated.

*Method of treatment*

The references to methods of treatment in this description are to be interpreted as references to the compounds, pharmaceutical compositions and medicaments of the present invention for use in a method for the treatment of the human (or animal) body by therapy (or for diagnosis).

The pharmaceutical formulations as disclosed herein are particularly effective for use in the treatment of ischemic and inflammatory conditions.

In one embodiment there is provided a liquid pharmaceutical formulation as disclosed herein for use as a medicament.

In one embodiment there is provided a liquid pharmaceutical formulation as disclosed herein for use in the treatment of an ischemic condition and/or an inflammatory condition.

In one embodiment there is provided the use of a liquid pharmaceutical formulation as disclosed herein for the manufacture of a medicament for use in the treatment of an ischemic condition and/or an inflammatory condition.

In one embodiment there is provided a method of treatment of an ischemic condition and/or an inflammatory condition, said method comprising one or more steps of administering a liquid pharmaceutical formulation as disclosed herein.

In one embodiment there is provided a liquid pharmaceutical formulation of pH > 6, said formulation comprising

a. a therapeutically effective amount of an Annexin A1 (AnxA1) N-terminal-peptide selected from the group consisting of

AMVSEFLKQAWFIENEEQEYVQTVKSSKGGPGSAVSPYPTFNPSSDVAA (SEQ ID NO:4; Annexin A1 2-50), AMVSEFLKQAWFIENEEQEYVQTVKSSKGGPGSAVSPYPTFNPSSOV (SEQ ID NO:5; Annexin A1 2-48), AMVSEFLKQAWFIENEEQEYVQTVKSSKGGPGSAVSPYPTFNPSS (SEQ ID NO:6; Annexin A1 2-46), AMVSEFLKQAWFIENEEQEYVQTLKSSKGGPGSAVSPYPTFNPSSOVAA (SEQ ID NO:7; Annexin A1 2-50 V24L), AMVSEFLKQAWFIENEEQEYVQT**L**KSSKGGPGSAVSPYPTFNPSSDV (SEQ ID NO:8; Annexin A1 2-48 V24L), and AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent selected from the group consisting of

i. water, such as ultrapure water; and
ii. a solution having an ionic strength of 1 $\mu$M to 50 mM,

wherein said solvent comprises one or more isotonicity modifiers selected from a carbohydrate and/or a sugar alcohol,
and optionally a buffer,

for use in the treatment of an ischemic condition and/or an inflammatory condition.

In one embodiment there is provided a liquid pharmaceutical formulation of pH $\geq$ 8; said formulation comprising

a. an Annexin A1 (AnxA1) N-terminal-peptide selected from the group consisting of

AMVSEFLKQAWFIENEEQEYVQTVKSSKGGPGSAVSPYPTFNPSSDV
AA (SEQ ID NO:4; Annexin A1 2-50),

AMVSEFLKQAWFIENEEQEYVQTVKSSKGGPGSAVSPYPTFNPSSOV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQT**L**KSSKGGPGSAVSPYPTFNPSSDV
AA (SEQ ID NO:7; Annexin A1 2-50 V24L),

AMVSEFLKQA WFI EN EEQEYVQTLKSSKGGPGSA VSPYPTFN PSSOV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L), and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent, wherein said solvent is a solution having an ionic strength of 1 $\mu$M to 50 mM,

and optionally a buffer,

for use in the treatment of an ischemic condition and/or an inflammatory condition.

In some embodiments of the present disclosure, said treatment is prophylactic, ameliorative or curative.

In some embodiments of the present disclosure, said ischemic condition and/or inflammatory condition is acute, subacute or chronic.

*Administration routes*

It will be appreciated that the preferred route of administration will depend on the general condition and age of the subject to be treated, the nature of the condition to be treated, the location of the tissue to be treated in the body and the active ingredient chosen.

In one embodiment of the present disclosure, the route of administration allows for introducing the pharmaceutical formulation comprising Annexin A1 (AnxA1) N-terminal-peptide into the blood stream to ultimately target the sites of desired action.

In one embodiment of the present disclosure the routes of administration is any suitable routes, such as an *enteral route* (including the oral, rectal, nasal, pulmonary, buccal, sublingual, transdermal, intracisternal and intraperitoneal administration), and/or a *parenteral route* (including subcutaneous, intramuscular, intrathecal, intravenous and intradermal administration). Appropriate dosage forms may be prepared by conventional techniques.

Parenteral administration is any administration route not being the oral/enteral route whereby the medicament avoids first-pass degradation in the liver. Accordingly, parenteral administration includes any injections and infusions, for example bolus injection or continuous infusion, such as intravenous administration, intramuscular administration or subcutaneous administration. Furthermore, parenteral administration includes inhalations and topical administration.

Accordingly, the pharmaceutical formulations according to the present disclosure is in one embodiment administered topically to cross any mucosal membrane of an animal to which the formulation is to be given, e.g. in the nose, vagina, eye, mouth, genital tract, lungs, gastrointestinal tract, or rectum, for example the mucosa of the nose, or mouth, and accordingly, parenteral administration may also include buccal, sublingual, nasal, rectal, vaginal and intraperitoneal administration as well as pulmonary and bronchial administration by inhalation or installation. In some embodiments, the formulations as disclosed herein are administered topically to cross the skin.

In one embodiment of the present disclosure, the intravenous, subcutaneous and intramuscular forms of parenteral administration are employed.

In one embodiment of the present disclosure, the pharmaceutical formulations as disclosed herein is used as a local treatment, i.e. is introduced directly to the site(s) of action. Accordingly, the pharmaceutical formulations may be applied to the skin or mucosa directly, or may be injected into the site of action, for example into the diseased tissue or to an end artery leading directly to the diseased tissue.

In a preferred embodiment the pharmaceutical formulations as disclosed herein is administered via the parenteral route. Preferred parenteral routes for administration of the pharmaceutical formulations of the present disclosure include intravenous, intramuscular, subcutaneous, intraperitoneal, intraarterial, nasal, pulmonary and buccal routes. Most preferred routes include intravenous, intraperitoneal, intramuscular and subcutaneous routes of administration.

*Administration and dosage*

According to the present disclosure, the pharmaceutical formulations comprising Annexin A1 N-terminal-peptides as provided herein are in one embodiment administered to individuals in need thereof in pharmaceutically effective doses or a therapeutically effective amount.

A therapeutically effective amount of an Annexin A1 N-terminal-peptide according to the present disclosure is in one embodiment an amount sufficient to cure, prevent, reduce the risk of, alleviate or partially arrest the clinical manifestations of a given disease or disorder and its complications. The amount that is effective for a particular therapeutic purpose will depend on the severity and the sort of the disorder as well as on the weight and general state of the subject. An amount adequate to accomplish this is defined as a "therapeutically effective amount".

In one embodiment the pharmaceutical formulation of the present disclosure is administered in doses of from 1 $\mu$g/day to 100 mg/day; such as from 1 $\mu$g/day to 10 $\mu$g/day, such as 10 $\mu$g/day to 100 $\mu$g/day, such as 100 $\mu$g/day to 250 $\mu$g/day, such as 250 $\mu$g/day to 500 $\mu$g/day, such as 500 $\mu$g/day to 750 $\mu$g/day, such as 750 $\mu$g/day to 1 mg/day, such as 1 mg/day to 2 mg/day, such as 2 mg/day to 5 mg/day, or such as 5 mg/day to 10 mg/day, such as 10 mg/day to 20 mg/day, such as 20 mg/day to 30 mg/day, such as 30 mg/day to 40 mg/day, such as 40 mg/day to 50 mg/day, such as 50 mg/day to 75 mg/day, or such as 75 mg/day to 100 mg/day.

In one embodiment of the present disclosure, one single dose of the pharmaceutical formulation of the present disclosure is administered and may comprise of from 1 μg/kg body weight to 100 mg/kg body weight; such as from 1 to 10 μg/kg body weight, such as 10 to 100 μg/day, such as 100 to 250 μg/kg body weight, such as 250 to 500 μg/kg body weight, such as 500 to 750 μg/kg body weight, such as 750 μg/kg body weight to 1 mg/kg body weight, such as 1 mg/kg body weight to 2 mg/kg body weight, such as 2 to 5 mg/kg body weight, such as 5 to 10 mg/kg body weight, such as 10 to 20 mg/kg body weight, such as 20 to 30 mg/kg body weight, such as 30 to 40 mg/kg body weight, such as 40 to 50 mg/kg body weight, such as 50 to 75 mg/kg body weight, or such as 75 to 100 mg/kg body weight.

In one embodiment, a dose according to the present disclosure is administered one or several times per day, such as from 1 to 6 times per day, such as from 1 to 5 times per day, such as from 1 to 4 times per day, such as from 1 to 3 times per day, such as from 1 to 2 times per day, such as from 2 to 4 times per day, such as from 2 to 3 times per day.

## Sequences

Full length Annexin A1 (homo sapiens) has the following sequence:

>sp|P04083|ANXA1_HUMAN Annexin A1 OS=Homo sapiens GN=ANXA1 PE=1 SV=2

MAMVSEFLKQAWFIENEEQEYVQTVKSSKGGPGSAVSPYPTFNPSSDVAALHKAIMV
KGVDEATIIDILTKRNNAQRQQIKAAYLQETGKPLDETLKKALTGHLEEVVLALLKTPAQ
FDADELRAAMKGLGTDEDTLIEILASRTNKEIRDINRVYREELKRDLAKDITSDTSGDFR
NALLSLAKGDRSEDFGVNEDLADSDARALYEAGERRKGTDVNVFNTILTTRSYPQLR
RVFQKYTKYSKHDMNKVLDLELKGDIEKCLTAIVKCATSKPAFFAEKLHQAMKGVGTR
HKALIRIMVSRSEIDMNDIKAFYQKMYGISLCQAILDETKGDYEKILVALCGGN (SEQ ID
NO:1).

MAMVSEFLKQ AWFIENEEQE YVQTVKSSKG GPGSAVSPYP TFNPSSDVAA LHKA (AnxA1 1-54; SEQ ID NO:2)

AMVSEFLKQ AWFIENEEQE YVQTVKSSKG GPGSAVSPYP TFNPSSDVAA LHKA (AnxA1 2-54; SEQ ID NO:3)

AMVSEFLKQAWFIENEEQEYVQTVKSSKGGPGSAVSPYPTFNPSSDVAA (SEQ ID NO:4; Annexin A1 2-50)

AMVSEFLKQAWFIENEEQEYVQTVKSSKGGPGSAVSPYPTFNPSSDV (SEQ ID NO:5; Annexin A1 2-48)

AMVSEFLKQAWFIENEEQEYVQTVKSSKGGPGSAVSPYPTFNPSS (SEQ ID NO:6; Annexin A1 2-46)

AMVSEFLKQAWFIENEEQEYVQTLKSSKGGPGSAVSPYPTFNPSSOVAA (SEQ ID NO:7; Annexin A1 2-50 V24L)

AMVSEFLKQAWFIENEEQEYVQT**L**KSSKGGPGSAVSPYPTFNPSSDV (SEQ ID NO:8; Annexin A1 2-48 V24L)

AMVSEFLKQAWFIENEEQEYVQTLKSSKGGPGSAVSPYPTFNPSS (SEQ ID NO:9; Annexin A1 2-46 V24L)

AMVSEFLKQAWFIENEEQEYVQTVK (SEQ ID NO:10; Annexin A1 2-26),

AMVSEFLKQAWFIENEEQEYVQTLK (SEQ ID NO:11; Annexin A1 2-26 V24L)

All peptides are in a preferred embodiment C-terminally amidated (-NH$_2$).

## Examples

### General materials and methods

In the following examples, "AnxA1" and reference to 'tested peptide' is identified herein as SEQ ID NO:8 (Annexin A1 2-48 V24L), AMVSEFLKQAWFIENEEQEYVQTLKSSKGGPGSAVSPYPTFNPSSDV, with a C-terminal amidation.

*Thioflavin T, "ThT"*

Thioflavin T (IUPAC: 4-(3,6-dimethyl-1,3-benzothiazol-3-ium-2-yl)-N,N-dimethylaniline chloride; CAS Number: 2390-54-7) hereafter known as ThT (the molecule) and ThT assay (the assay).

ThT is widely used to visualize and quantify the presence of misfolded peptide and protein aggregates called amyloids or fibrils. ThT is a fluorescent molecule that binds to inter molecular beta-sheets in peptides and proteins (here AnxA1). When excited around 450 nm ThT exhibits a stronger fluorescence intensity when bound to amyloids and fibrils. An increase in fluorescence indicates that more ThT molecules are bound to the peptide this again indicates that more peptide is misfolded as an amyloid or fibril. A weaker fluorescence intensity is indicative of less amyloids or fibrils present with fewer intermolecular beta-sheets where ThT can bind.

*Thioflavin T assay, "ThT assay"*

Some peptides have a tendency to form fibrils and amyloids and the propensity is dependent on the formulation conditions in an unpredictable manor. Fibrillation is a stochastic process and to avoid long analysis times and both false negatives and positive results, shear stress is introduced to the sample by rigorous rotation in microtiter plates while continuously monitoring the fluorescence emission at approximately 486 nm. An increase in the fluorescence is an indication of the formation of amyloids or fibrils and the time it takes for the fluorescence to start increasing is known as the lag-time. A faster (short) lag-time is an indication of higher propensity for the peptide to degrade by formation of fibrils or amyloids.

A ThT fluorescence of 50 AU at t=0 is considered noise or background. Lag time is reported as the time it takes for the signal intensity to exceed 50 AU.

All samples for the ThT assay were filtered through cellulose acetate filters (22 $\mu$m cutoff, $\varnothing$ = 13 mm, rinsed with MilliQ® water prior to filtration). For each sample, 20 $\mu$L ThT of a 1 mM solution was added to 1 mL peptide solution. From this sample mixture, 200 $\mu$L was transferred into n wells in a 96 well microtiter plate (n = 3-6). 96-well NUNC plates Bottom optics and Sealing tape, Thermo Scientific™ Nunc™ Sealing Tapes, was used. Lag-time is reported as an average of n samples.

A fluorescence plate reader (CLARIOstar® Plus, BMG Labtech) was used for the experiments with the following setting: Excitation wavelength 450 nm; Dichroic filter 465 nm; Emission wavelength 486 nm; Focal height 3.5 mm; Gain 1000; Number of cycles 960; Cycle time 360 s; Number of flashes per well 20; Temperature 25°C; Shake mode orbital, Frequency of 300 rpm, 200 s, and shake after each cycle.

*ThT assay - lag-time*

If possible, the average of all ThT data replicates are used for ThT lag-time determination. In case of very stochastic data, worst-case data is used for lag-time evaluation. ThT lag-time was determined using either a 1-step or 2-step sigmoidal curve. In cases where the fibrillation did not follow a sigmoidal curve or did not reach plateau within the experimental time, the lag-time was evaluated as the time it takes for the signal to exceed 50 AU.

*Determination of concentration using UV-VIS*

The concentration of AnxA1 was determined using UV-VIS spectroscopy using a Nanodrop 2000c from Thermo scientific and an extinction coefficient of $A_{280}^{0.1\%} = 1.64$ ml mg$^{-1}$cm$^{-1}$.

*Dissolution time*

The peptide was initially wetted with a small amount of solvent and left for 5 minutes and following added the full amount of solvent. The solution was swirled by hand and observation of final dissolution time noted.

The solutions intended for lyophilization were dissolved by wetting and following added the rest of the solution. Due to the large volume the five formulations were left on a slow rocker for dissolution.

*Lyophilization*

Samples were prepared prior to lyophilization and stored at 80°C overnight. The samples were prepared with 2 mL AnxA1 solutions in 6R vials, (clear Fiolax, Such & Holm) with stoppers (20 mm Lyo NovaPure, West Pharma). Samples were placed in the freeze dryer (Telstar LyoQuest, Azbil Telstar Technologies S.L.U.) pre-cooled to - 55°C. The condenser temperature was kept at -55°C and the pressure at 0.2 mbar for 48 hours. Formulation 1-5 vial 1-5 were placed on the middle shelf in the tower and formulation 1-5 vial 6-10 were placed on the lower shelf. The tower tube (22Ø, 25 cm tall) is placed outside the condenser at ambient temperature with the lower shelf closest to the condenser.

### Example 1

All samples contained 0.9 % NaCl (154 mM) in double deionized (Milli Q®) water with pH ranging from 7.1 to 7.4. The concentration of AnxA1 was between 1.6 mg/mL to 20 mg/mL according to table 1.

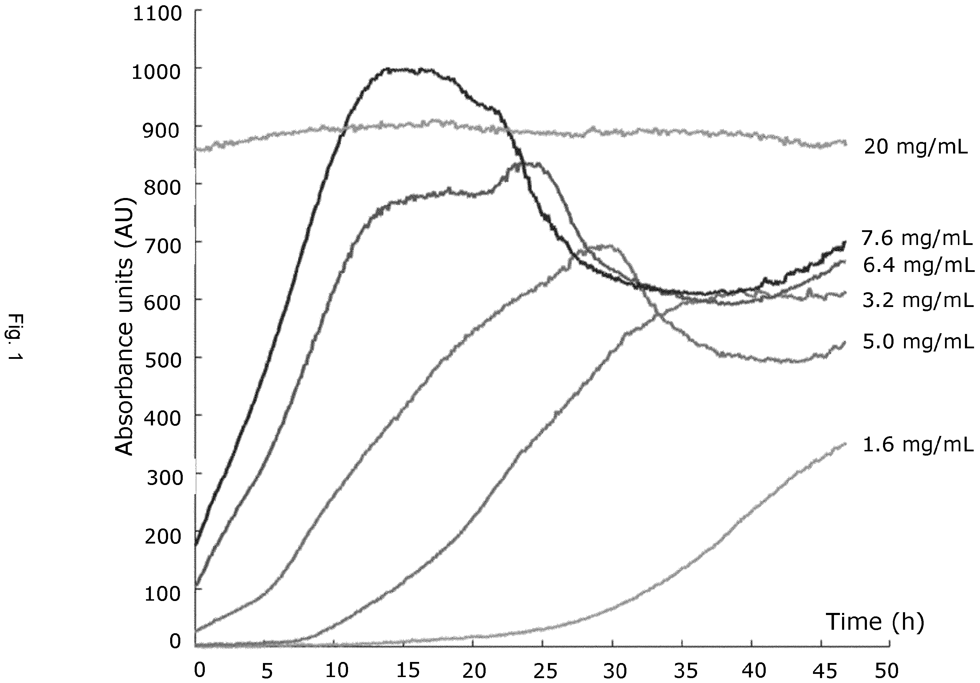

The data in figure 1 and table 1 shows the results of the ThT assay for AnxA1 at different concentrations with 0.9% NaCl in Milli Q® water. The data clearly shows that at around neutral pH the peptide degrades by the formation of amyloids or fibrils in a concentration dependent manner. At concentrations ≥ 5 mg/mL AnxA1 has already initiated the degradation (fibrillation) before the analysis is started indicating very high fibrillation propensity.

Table 1: sample composition and lag-time for ThT assay indicating a reverse relationship between concentration and stability from the increase in lag-time when concentration is lower.

| AnxA1 (mg/mL) | NaCl (%) | pH | Avg. lag-time (h) |
|---|---|---|---|
| 1.6 | 0.9 | 7.1 | 28.3 |
| 3.2 | 0.9 | 7.4 | 9.3 |
| 5.0 | 0.9 | 7.3 | 2.7 |
| 6.4 | 0.9 | 7.3 | 0 |
| 7.6 | 0.9 | 7.3 | 0 |
| 20 mg/mL* | 0.9 | 8.1 ** | 0 |
| *The sample concentration was not determined due to gel-like texture. 20 mg/mL is estimated from the amount weighed into the sample. <br> ** pH value determined is not considered valid due to the gel-like texture of the sample. | | | |

### Example 2

20 mg freeze-dried AnxA1 was added per mL of 0.9% NaCl (154 mM) in Milli Q® at neutral pH and the peptide was not readily dissolved. The peptide suspension was left over night at room temperature on rotation and the next morning the emulsion has turned into an opaque gel like substance. An aliquot was transferred to an Eppendorf tube, spun down and the concentration in the supernatant determined by UV-VIS to approximately 0.17 mg/mL.

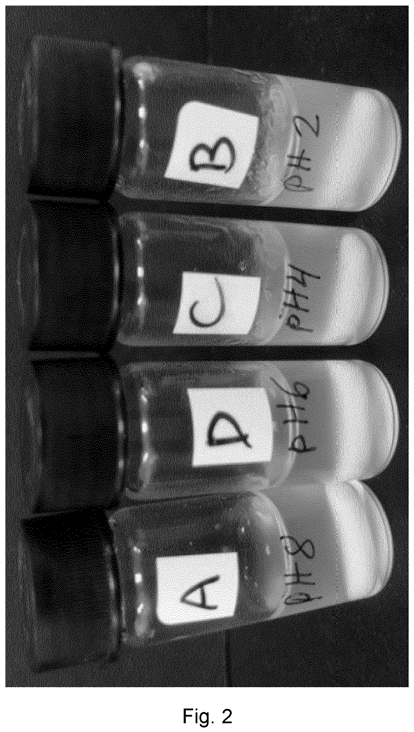

The remainder of the peptide suspension was diluted to an assumed concentration of 5 mg/mL and 4*1 mL samples were adjusted to pH 2, 4, 6 and 8, (see Figure 2). All 4 vials continued to be opaque indicating limited solubility.

### Example 3

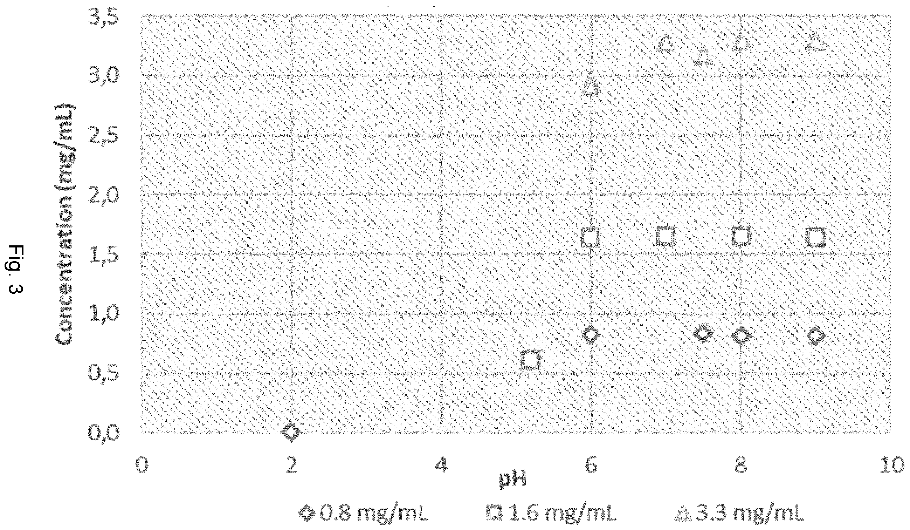

Three samples of AnxA1 was prepared at pH 9 in Milli Q® water, 0.9 % NaCl (154 mM) at concentrations 3.3, 1.6 and 0.8 mg/mL, respectively. pH was adjusted by addition of 0.1 M NaOH to each of the samples and the concentration determined after centrifugation using UV-VIS spectroscopy. At pH 6.0 the samples became opaque and precipitated immediately below pH 6.0 (cf. Fig 3).

### Example 4

1.6 or 3.2 mg AnxA1 was added to Milli Q® water at around neutral pH containing different salts (NaCl, $CaCl_2$, $ZnCl_2$ or HCl) according to table 2. Dissolution times was observed and the resulting solutions was analyzed using the ThT assay.

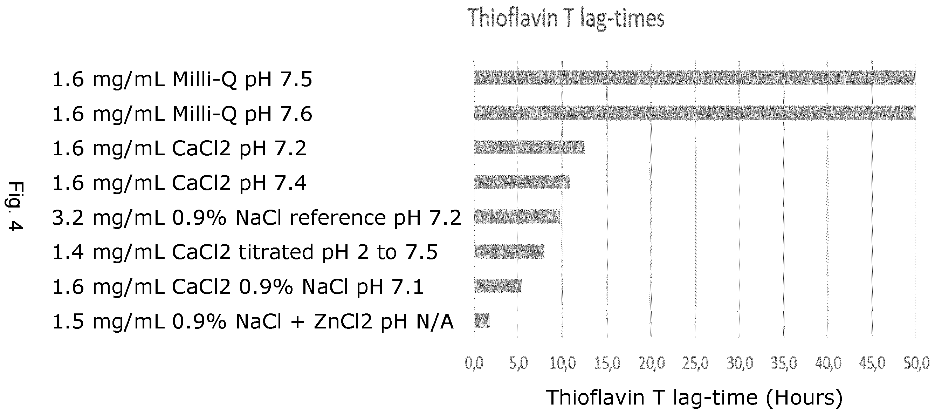

It is clearly seen that the dissolution time is much faster at low salt concentrations and, at the same time, the lag-time in the ThT assay is longer indicating less propensity to degradation via amyloid or fibril formation. The best stability is observed with no addition of salt where dissolution is less than 5 minutes and lag-time > 50 hours. Neither NaCl, $CaCl_2$ nor $ZnCl_2$ decreased the propensity of AnxA1 to degrade via amyloid or fibril formation (cf. Fig. 4).

**Table 2:** Seven samples of AnxA1 in Milli Q® water at around neutral pH containing different salts (NaCl, CaCl2, ZnCl$_2$ or HCl). It is clearly seen that the dissolution time is much faster at low salt concentrations and at the same time the lag-time in the ThT assay is longer. The best stability is observed with no addition of salt where dissolution is less than 5 minutes and lag-time > 50 hours (fibrillation not observed within the maximum assay time).

| Anx01 (mg/mL) | NaCl (%) | CaCl$_2$ (mM) | ZnCl$_2$ (mM) | HCl (mM) | pH | Dissolution time (Notes) | Avg. lag-time (h) |
|---|---|---|---|---|---|---|---|
| 1.6 | - | - | - | - | 7.6 | 5 min | > 50.0* |
| 1.6 | - | - | - | - | 7.5 | 5 min | > 50.0* |
| 1.6 | - | 0.77 | - | - | 7.2 | 5 min | 12.5 |
| 1.6 | - | 0.77 | - | - | 7.4 | 10 min | 10.8 |
| 3.2 | 0.9 | - | - | - | 7.2 | 45 min | 9.7 |
| 1.6 | 0.9 | 0.77 | - | - | 7.1 | 35 min | 5.4 |
| 1.5 | 0.9 | - | 0.77 | - | N/A | not fully solubilized after more than 2 hours | 1.8 |
| * No increase in fluorescence observed within the timeframe of the analysis (50 hours) | | | | | | | |

### Example 5

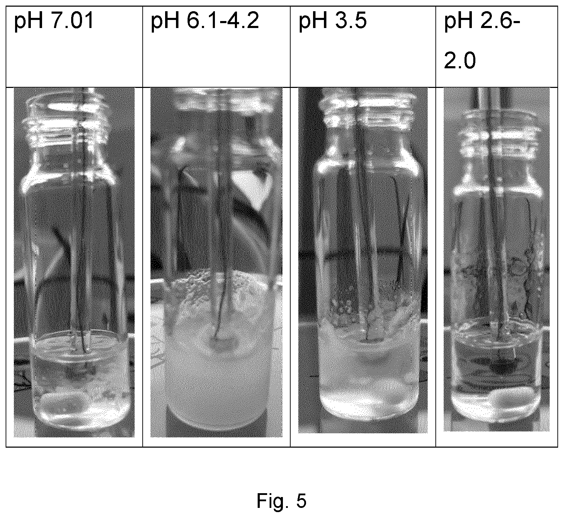

Approximately 16 mg/mL AnxA1 was added to milliQ® water. The resulting pH was 7.5. pH was adjusted to pH 10 in one sample using 0.1 M NaOH and to pH 2 using 0.1 M HCl in another sample, according to table 3. The appearance of the solution was noted. At pH $\geq$ 7.4 the solution was clear. At pH $\leq$ 7.1 > 2.6 solutions where unclear. At pH $\leq$ 2.6 the solution was again clear. Data are summarized the Table 3, see also Figure 5.

**Table 3**: Visual appearance of samples containing AnxA1 at different pH values. A clear solution was observed $\geq$ 7.4 and from 2.6 - pH 2.0.

| pH | Description |
|---|---|
| $\geq$ 7.4 | Clear |
| 7.1 | Fluffy particles |
| 6.8-6.3 | Milky, turbid |
| 6.1-4.2 | Gel-like |
| 3.8-3.1 | Starts clearing up |
| 2.6-2.0 | Clear solution |

### Example 6

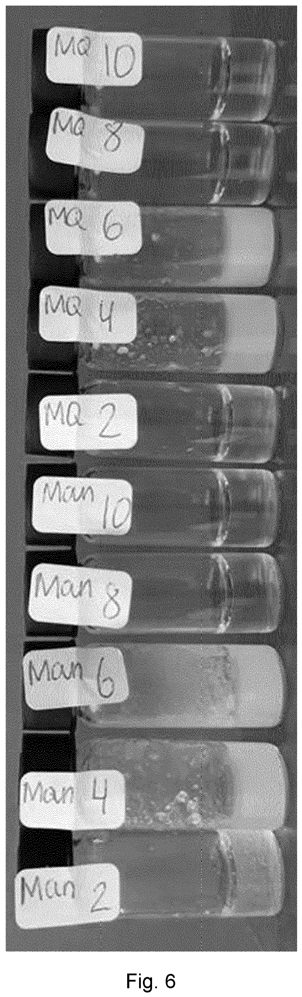

AnxA1 samples are dissolved in Milli Q® water or 5% mannitol in Milli Q® water to approximately 18 mg/mL and pH was adjusted using 1.0 M HCl and NaOH. After dissolution visual appearance was noted and a photograph was taken, see Figure 6 and Table 4, and a sample was cleared by centrifugation and the concentration determined in the supernatant using UV-VIS spectroscopy. A clear solution was observed at pH 8 and 10 both with and without 5% Mannitol in Milli Q® water. At pH 2, 4 and 6 an unclear and or viscous solution was observed according to Table 4 and Figure 6.

**Table 4**: Concentration of AnxA1 and visual appearance of solution in Milli Q® water with and without mannitol from pH 2 to 10. A clear solution is observed at pH 8 and 10

| Sample | Sample name in figure 6 | pH | Concentration (mg/ml) | Description |
|---|---|---|---|---|
| Mannitol 5% | Man 2 | 2.1 | 15.5 | Not fully soluble, Unclear, viscous texture |
| Mannitol 5% | Man 4 | 4.0 | 0.4 | Not fully soluble, Unclear/milky, gel-like |
| Mannitol 5% | Man 6 | 6.0 | 0.8 | Not fully soluble, Unclear/milky |

(continued)

| Sample | Sample name in figure 6 | pH | Concentration (mg/ml) | Description |
|---|---|---|---|---|
| Mannitol 5% | Man 8 | 8.0 | 16.9 | Clear |
| Mannitol 5% | Man 10 | 10.1 | 17.6 | Clear |
| Milli-Q® | MQ 2 | 2.1 | 15.9 | Not fully soluble, Clear with viscous texture |
| Milli-Q® | MQ 4 | 4.0 | 0.6 | Not fully soluble, Unclear/milky, gel-like |
| Milli-Q® | MQ 6 | 6.0 | 1.9 | Not fully soluble, Unclear/milky |
| Milli-Q® | MQ 8 | 8.1 | 17.0 | Clear |
| Milli-Q® | MQ 10 | 10.1 | 17.6 | Clear |

### Example 7

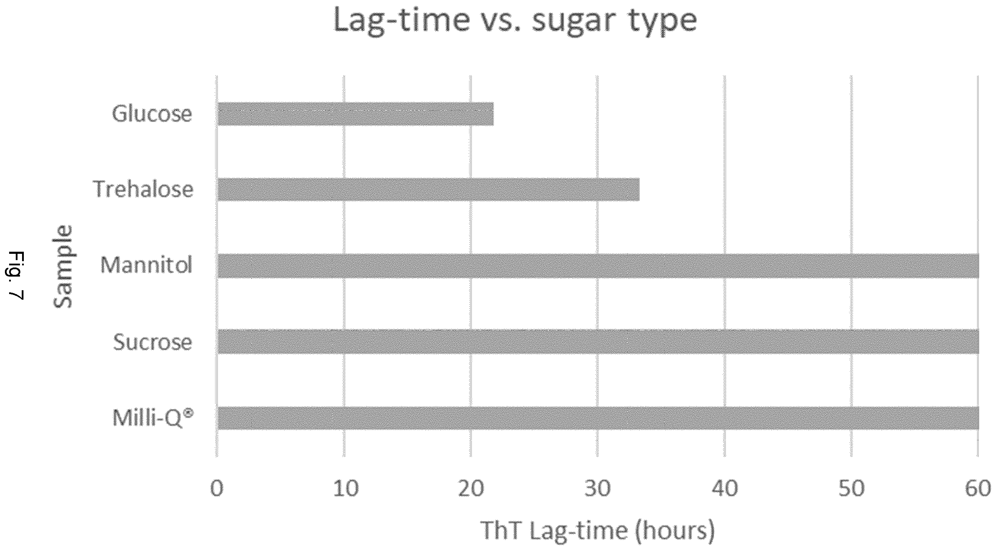

Isotonic samples containing 1.5 - 1.6 mg/mL AnxA1 and a tonicity provider (isotonicity modifier) was prepared around neutral pH according to the specification in table 5. All samples were observed as clear solutions. The samples were subjected to the ThT assay previously described, where a higher lag-time was observed when using sucrose and mannitol as tonicity providers compared to using trehalose and glucose. The data presented in Table 5 is illustrated graphically in Figure 7.

Table 5: Isotonic samples containing 1.5 - 1.6 mg/mL AnxA1 was prepared around neutral pH according to the specification presented herein.

| AnxA1 (mg/mL) | Glucose (wt.%) | Sucrose (wt.%) | Mannitol (wt.%) | Trehalose (wt.%) | pH | Avglag time (h) | Visual appearance |
|---|---|---|---|---|---|---|---|
| 1.5 | 5 | - | - | - | 7.1 | 21.7 | Clear |
| 1.6 | - | 10 | - | - | 7.2 | 65* | Clear |
| 1.6 | - | | 5 | - | 6.7 | 65* | Clear |
| 1.6 | - | - | - | 10 | 7.2 | 33.3 | Clear |
| *No increase in fluorescence observed within the timeframe of the analysis (65 hours) | | | | | | | |

### Example 8

Samples containing AnxA1 at different concentrations were prepared in Milli Q® water according to table 6. A reference sample was prepared in Saline, 0.9% NaCl (154 mM NaCl). The lag-time was determined using the ThT assay previously described. In Milli Q® water it is clear that higher degradation propensity via amyloid or fibril formation is observed at higher concentration of AnxA1. It is additionally observed that all solutions of AnxA1 in Milli Q water are more stable than the reference sample in saline (154 mM NaCl).

Table 6: Lag-time data determined using the ThT assay of AnxA1 samples in Milli Q® water at different concentrations compared to an Axn01 sample in 0.9% (154 mM) NaCl. All samples had a pH of 7.4

| AnxA1 (mg/mL) | Solubilized in | pH | Avg. lag-time (h) |
|---|---|---|---|
| 1.7 | Milli-Q® water | 7.4 | 65* |
| 3.1 | Milli-Q® water | 7.4 | 50.4 |
| 3.2 | Saline (0.9% NaCl) | 7.4 | 5.6 |
| 4.6 | Milli-Q® water | 7.4 | 61.4 |
| 6.1 | Milli-Q® water | 7.4 | 29.6 |
| 8.0 | Milli-Q® water | 7.4 | 11.5 |
| *No increase in fluorescence observed within the timeframe of the analysis (65 hours) | | | |

*Example 9*

ThT assay was used to assess the physical stability (fibrillation propensity) of AnxA1. The physical stability was tested in glycyl-glycine buffer pH 8.0 and tris buffer pH 7.4 with either sucrose, mannitol or sorbitol as isotonicity modifier to provide an isotonic solution, the concentration of AnxA1was between 1.5 mg/mL and 4.8 mg/mL

The Lag-time is > 65 hours (the ThT assay is running) in all samples containing 10 mM glycyl-glycine, pH 8 independent of the concentration of AnxA1 and isotonicity modifier.

In 10 mM tris, pH 7.5, fibrillation is observed after 10.7 to 11.1 hours in all samples at the higher concentrations (4.7 mg/mL). At lower concentration 1.5 mg/mL in 10% sucrose the lag-time is 42.8 hours and at 1.7 mg/mL containing 5% mannitol or sorbitol the lag-time is > 65 hours.

**Table 7:**

| AnxA1 (mg/mL ) | Isotonic ity provide r | Mann. (wt.%) | Sorb. (wt.%) | Sucr. (wt.%) | Hist. (mM) | Tris (mM) | Glycyl glycin e (mM) | pH | Lag-time (hours) |
|---|---|---|---|---|---|---|---|---|---|
| 4.8 | Mannitol | 5 | - | - | 10 | - | - | 6.6 | 42.1 |
| 4.8 | Sorbitol | - | 5 | - | 10 | - | - | 6.7 | 21.3 |
| 4.8 | Sucrose | - | - | 10 | 10 | - | - | 6.6 | 48.8 |
| 1.7 | Mannitol | 5 | - | - | - | 10 | - | 7.5 | 65* |
| 1.7 | Sorbitol | - | 5 | - | - | 10 | - | 7.6 | 65* |
| 1.5 | Sucrose | - | - | 10 | - | 10 | - | 7.5 | 42.8 |
| 4.7 | Mannitol | 5 | - | - | - | 10 | - | 7.5 | 10.7 |
| 4.7 | Sorbitol | - | 5 | - | - | 10 | - | 7.5 | 10.8 |
| 4.7 | Sucrose | - | - | 10 | - | 10 | - | 7.5 | 11.1 |
| 1.6 | Mannitol | 5 | - | - | - | - | 10 | 8.0 | 65* |
| 1.7 | Sorbitol | - | 5 | - | - | - | 10 | 8.0 | 65* |
| 1.6 | Sucrose | - | - | 10 | - | - | 10 | 8.0 | 65* |
| 3.1 | Mannitol | 5 | - | - | - | - | 10 | 8.0 | 65* |
| 3.2 | Mannitol | 5 | - | - | - | - | 10 | 8.0 | 65* |
| 3.3 | Mannitol | 5 | - | - | - | - | 10 | 8.0 | 65* |
| 4.7 | Mannitol | 5 | - | - | - | - | 10 | 8.0 | 65* |
| 4.6 | Sorbitol | - | 5 | - | - | - | 10 | 8.0 | 65* |
| 4.8 | Sucrose | - | - | 10 | - | - | 10 | 7.9 | 65* |
| Mann.: Mannitol, Sorb.: Sorbitol, Sucr.: Sucrose, Hist.: Histidine, Tris: Tris-buffered saline (TBS). *No increase in fluorescence observed within the timeframe of the analysis (65 hours) | | | | | | | | | |

*Example 10*

The ThT assay was used to assess the physical stability and fibrillation propensity of AnxA1. A design of experiment was performed according to table 8 in 10-50 mM glycyl-glycine buffer, pH 7.4 -8.5, with sucrose, mannitol or mannitol/sucrose at AnxA1 concentrations between 1.5 and 8.4 mg/mL.

The physical stability of AnxA1 increases (longer lag-time) with increasing pH from 7.4 to 8.5. The physical stability decreases (shorter lag-time) with increasing glycyl-glycine buffer concentration from 10 to 50 mM. The effect of the isotonicity modifier used (mannitol, sucrose and mannitol/sucrose) had a negligible effect on the physical stability (lag-time).

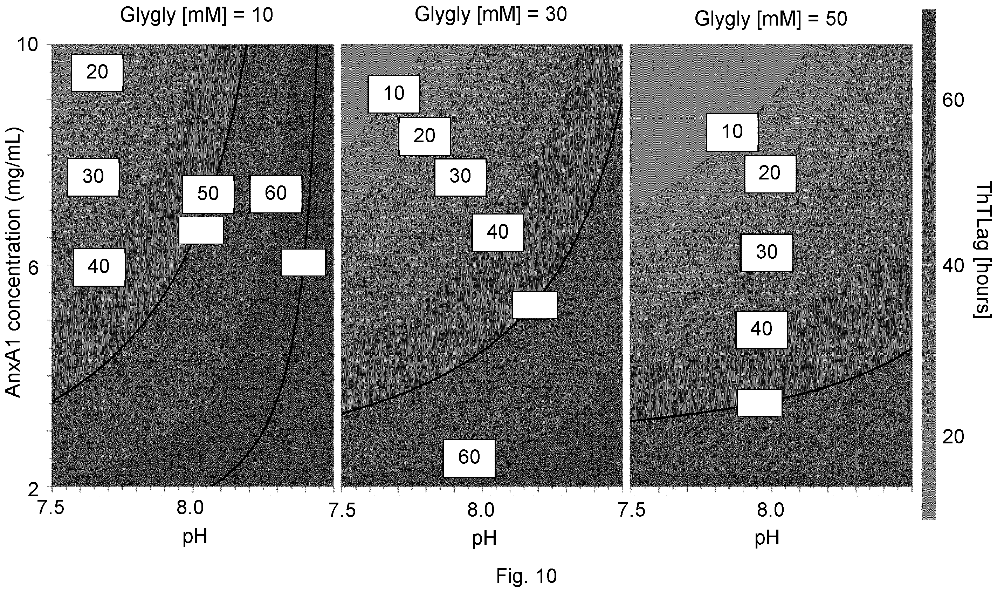

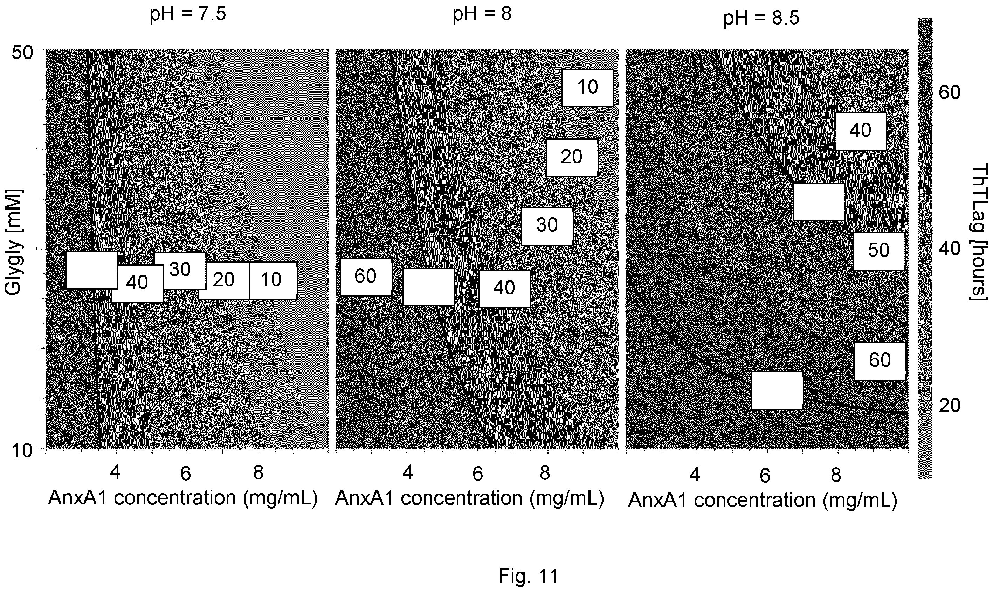

Based on the experimental data from the DoE the effect of buffer concentration, AnxA1 concentration and pH on lag-time was modeled and plotted in three dimensional contour plots in figure 10 and figure 11.

**Table 8:**

| AnxA1 concentrati on (mg/mL) | Mann. (wt. %) | Sucr. (wt. %) | Glycylglycine (mM) | pH | Lag-time (hours) |
|---|---|---|---|---|---|
| 1.5 | 5 | - | 10 | 7.4 | >65* |
| 8.1 | 5 | - | 10 | 7.4 | 13.7 |
| 1.5 | 5 | - | 50 | 7.5 | >65 |
| 8.0 | 5 | - | 50 | 7.5 | 3.1 |
| 1.5 | 4 | 2.5 | 10 | 7.5 | >65 |
| 8.0 | 4 | 2.5 | 10 | 7.5 | 15.1 |
| 7.9 | - | 10 | 10 | 7.5 | 23.8 |
| 1.5 | 4 | 2.5 | 50 | 7.6 | >65 |
| 8.0 | 4 | 2.5 | 50 | 7.6 | 3.3 |
| 1.6 | - | 10 | 50 | 7.6 | 64.7 |
| 4.7 | - | 10 | 30 | 8.0 | >65 |
| 4.6 | - | 10 | 30 | 8.0 | 39 |
| 4.7 | - | 10 | 30 | 8.0 | 50 |
| 4.0 | 5 | - | 10 | 8.2 | >65 |
| 8.4 | 5 | - | 10 | 8.2 | 41 |
| 8.1 | - | 10 | 10 | 8.2 | >65 |
| 1.6 | 5 | - | e | 8.3 | >65 |
| 8.0 | 4 | 2.5 | 10 | 8.3 | >65 |
| 1.5 | - | 10 | 10 | 8.3 | >65 |
| 1.6 | 4 | 2.5 | 10 | 8.4 | >65 |
| 8.0 | 5 | - | 50 | 8.5 | 41.7 |
| 1.4 | 4 | 2.5 | 50 | 8.5 | >65 |
| 7.8 | 4 | 2.5 | 50 | 8.5 | 31.5 |
| 1.4 | - | 10 | 50 | 8.5 | >65 |
| 7.7 | - | 10 | 50 | 8.5 | 29.5 |
| Mann.: Mannitol, Sucr.: Sucrose. | | | | | |
| *No increase in fluorescence observed within the timeframe of the analysis (65 hours) | | | | | |

*Example 11*

Accelerated stability study.

Five formulations (Form 1-5) of AnxA1 was prepared as presented in table 9. Samples were lyophilized according to the procedure previously described. The chemical stability of the samples was determined as purity of AnxA1 by RP-HPLC (Reversed phase HPLC) in a 6 month stability study at 5$^O$C and 40°C according to table 10.

AnxA1 is observed to be physically stable (lag-time > 60 hours) in samples of all compositions at pH 8.3 (formulation 1, 2, 3 & 5). At pH 7.5 the physical stability is less as observed by the shorter lag-time of 34.7 hours. In saline (0.9 wt% NaCl) and Pbs buffer the physical stability is lower as observed from the short lag-times of 8.8 and 5.6 hours, respectively.

AnxA1 is observed to be more chemically stable (higher purity) in samples with a combination of mannitol and sucrose as isotonicity modifiers (Form 2, Form 3, Form 4 and Form 5) in comparison to the sample having only mannitol as an isotonicity modifier (Form 1). Sucrose thus increases chemical stability of AnxA1 in the freeze-dried cake.

**Table 9:**

| | Tween 80 (%) | Glygly (mM) | Sucr. (wt%) | Mann. (wt%) | NaCl (Wt%) |
|---|---|---|---|---|---|
| **Form 1** | - | 10 | 0 | 5 | - |
| **Form 2** | - | 10 | 1 | 4 | - |
| **Form 3** | - | - | 1 | 4 | - |
| **Form 4** | - | 10 | 1 | 4 | - |
| **Form 5** | 0.01 | 10 | 1 | 4 | - |
| **Saline** | - | - | - | - | 0.9 |
| **Pbs*** | - | - | - | - | - |
| ***Cont.*** | **Peptide (mg/mL)** | **pH** | **ThT Lag time (hrs)** | **Remarks** | |
| **Form 1** | 5 | 8.3 | >60 | - | |
| **Form 2** | 5 | 8.3 | >60 | - | |
| **Form 3** | 5 | 8.3 | >60 | - | |
| **Form 4** | 5 | 7.5 | 34.7 | - | |
| **Form 5** | 5 | 8.3 | >60 | - | |
| **Saline** | 3.6 | - | 8.8 | Reference for ThT assay | |
| **Pbs*** | 4.6 | 7.4 | 5.6 | Reference for ThT assay | |
| Glygly: Glycylglycine, Sucr.: Sucrose, Mann.: Mannitol. *Dulbecco's phosphate buffered saline from Merck. | | | | | |

**Table 10:**

| | Temp | t0 | 1M | 2M | 3M | 6M |
|---|---|---|---|---|---|---|
| Form 1 | 5 | 97,3 | | | 96,4 | |
| | 40 | 97,3 | 92,4 | 89,0 | 82,9 | 79,3 |
| Form 2 | 5 | 97,3 | | | 96,6 | |
| | 40 | 97,3 | 97,2 | 96,8 | 95,9 | 94,9 |
| Form 3 | 5 | 97,3 | | | 96,6 | |
| | 40 | 97,3 | 97,2 | 96,8 | 96,0 | 95,5 |
| Form 4 | 5 | 97,3 | | | 96,7 | |
| | 40 | 97,3 | 97,2 | 96,8 | 96,1 | 95,5 |
| Form 5 | 5 | 97,1 | | | 96,7 | |
| | 40 | 97,1 | 96,8 | 96,3 | 95,7 | 93,9 |

*Example 12*

Stability at hypertonic and hypotonic solutions.

Nine formulations of AnxA1 was prepared as presented in table 11 below, including a positive control for the ThT assay. The samples were analysed using the ThT assay using the procedures previously described with n=3 for samples containing $NaHCO_3$ and n=4 for samples containing glycylglycine.

Fibrillation is observed only in the positive control sample in 0.9% saline. No fibrillation was observed neither in $NaHCO_3$ nor in glycylglycine buffer at 20% hypotonicity, and at 20% and 100% hypertonicity.

**Table 11:**

| AnxA1 (mg/mL) | Tonicity (%) | NaCl (wt %) | Mann. (wt.%) | Sucr. (wt.%) | NaHCO$_3$ (mM) | Glygly mM | pH | Lag-time (hours) |
|---|---|---|---|---|---|---|---|---|
| 4 | 100 | 0.9 | - | - | - | - | - | 8.2* |
| 5 | 80 | - | 3.2 | 0.8 | - | 10 | 8.40 | < 60 |
| 5 | 120 | - | 4.8 | 1.2 | - | 10 | 8.41 | < 60 |
| 5 | 200 | - | 8.0 | 2.0 | - | 10 | 8.40 | < 60 |
| 5 | 100 | - | 4.0 | 1.0 | - | 10 | 8.39 | < 60 |
| 5 | 100 | - | 4.0 | 1.0 | 10 | - | 8.40 | < 60 |
| 5 | 80 | - | 3.2 | 0.8 | 10 | - | 8.41 | < 60 |
| 5 | 120 | - | 4.8 | 1.2 | 10 | - | 8.40 | < 60 |
| 5 | 200 | - | 8.0 | 2.0 | 10 | - | 8.44 | < 60 |
| Glygly: Glycylglycine, Sucr.: Sucrose, Mann.: Mannitol. *Positive control sample in 0,9 % saline | | | | | | | | |

**Claims**

1. A liquid pharmaceutical formulation of pH > 6,0; said formulation comprising

a. an Annexin A1 (AnxA1) N-terminal-peptide having an amino acid sequence selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSDV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV

AA (SEQ ID NO:7; Annexin A1 2-50 V24L),

AMVSEFLKQAWFIENEEQEYVQTLKSSKGGPGSAVSPYPTFNPSSDV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L),
and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent selected from the group consisting of

i. water, such as ultrapure water; and
ii. a solution having an ionic strength of 1 μM to 50 mM,

wherein said solvent comprises one or more isotonicity modifiers selected from a carbohydrate and/or a sugar alcohol.

2. A liquid pharmaceutical formulation of pH ≥ 8; said formulation comprising

a. an Annexin A1 (AnxA1) N-terminal-peptide having an amino acid sequence selected from the group consisting of

AMVSEFLKQAWFIENEEQEYVQTVKSSKGGPGSAVSPYPTFNPSSDV

AA (SEQ ID NO:4; Annexin A1 2-50),

AMVSEFLKQAWFIENEEQEYVQTVKSSKGGPGSAVSPYPTFNPSSDV (SEQ ID NO:5; Annexin A1 2-48),
AMVSEFLKQAWFIENEEQEYVQTVKSSKGGPGSAVSPYPTFNPSS (SEQ ID NO:6; Annexin A1 2-46),

AMVSEFLKQAWFIENEEQEYVQTLKSSKGGPGSAVSPYPTFNPSSDV

AA (SEQ ID NO:7; Annexin A1 2-50 V24L),

AMVSEFLKQAWFIENEEQEYVQTLKSSKGGPGSAVSPYPTFNPSSDV (SEQ ID NO:8; Annexin A1 2-48 V24L), and
AMVSEFLKQAWFIENEEQEYVQTLKSSKGGPGSAVSPYPTFNPSS (SEQ ID NO:9; Annexin A1 2-46 V24L),

and a variant of any one of SEQ ID NOs:4-9 comprising 1 to 6 individual amino acid substitutions, wherein said variant is a ligand and/or agonist of one or more of Formyl Peptide Receptor 1 (FPR1), Formyl Peptide Receptor 2 (FPR2) and Formyl Peptide Receptor 3 (FPR3); and

b. a solvent, wherein said solvent is a solution having an ionic strength of 1 $\mu$M to 50 mM.

3. The liquid pharmaceutical formulation according to claim 1, wherein pH $\geq$ 7,4, such as pH $\geq$ 7,5, such as pH $\geq$ 7,6, such as pH $\geq$ 7,7, such as pH $\geq$ 7,8, such as pH $\geq$ 7,9, such as pH $\geq$ 8,0, such as pH $\geq$ 8,3.

4. The liquid pharmaceutical formulation according to any of the preceding claims, wherein pH is 8 to 8,5.

5. The liquid pharmaceutical formulation according to any of the preceding claims, wherein the Annexin A1 N-terminal-peptide has an aqueous solubility of at least 0,1 mg/ml, such as at least 0,5 mg/ml, such as at least 1,0 mg/ml, such as at least 1,5 mg/ml, such as at least 2,0 mg/ml, such as at least 2,5 mg/ml, such as at least 3,0 mg/ml, such as at least 3,5 mg/ml, such as at least 4,0 mg/ml, such as at least 5,5 mg/ml, such as at least 5,0 mg/ml, such as at least 7,5 mg/ml, such as at least 10,0 mg/ml,
and/or said formulation having a concentration of said Annexin A1 N-terminal-peptide of 0,1 to 10 mg/ml, such as 0,1 to 0,5 mg/ml, such as 0,5 to 1 mg/ml, such as 1 to 2 mg/ml, such as 2 to 3 mg/ml, such as 3 to 4 mg/ml, such as 4 to 5 mg/ml, such as 5 to 6 mg/ml, such as 6 to 7 mg/ml, such as 7 to 8 mg/ml, such as 8 to 9 mg/ml, such as 9 to 10 mg/ml.

6. The liquid pharmaceutical formulation according to any of claims 2-5, wherein said solvent comprises one or more isotonicity modifiers, such as a carbohydrate and/or a sugar alcohol.

7. The liquid pharmaceutical formulation according to any of the preceding claims, wherein said isotonicity modifier is selected from the group consisting of pentose monosaccharides, hexose monosaccharides, disaccharides and trisaccharides,
and/or wherein said isotonicity modifier is selected from the group consisting of: sucrose, trehalose, mannose, ribose, maltose, glucose, lactose, galactose, arabinose, mannitol, sorbitol, inositol, glycerol, xylitol, propylene glycol, polyethylene glycols (PEGs) and polypropylene/ ethylene glycol copolymer.

8. The liquid pharmaceutical formulation according to any of the preceding claims, wherein the solution comprises sucrose and/or mannitol,
optionally wherein the solution comprises approx. 4% (w/v) mannitol and/or approx. 1% sucrose, such as comprises 3 to 5% (w/v) mannitol and/or 0,5 to 2% (w/v) sucrose, such as comprises 0 to 8 % (w/v) mannitol and/or 0 to 5% (w/v) sucrose.

9. The liquid pharmaceutical formulation according to any of the preceding claims further comprising one or more buffers,
such as one or more buffers selected from the group consisting of an acetate buffer, TRIS, amine-containing buffers, amino-acid based buffers such as lysine, hydroxy-lysine, histidine and glycyl-glycine, non-phosphate buffers, bicarbonate buffers, Polysorbate-80 (e.g. Tween 80), HEPES and borate.

10. The liquid pharmaceutical formulation according to any of the preceding claims, wherein said Annexin A1 N-terminal-peptide has the amino acid sequence AMVSEFLKQAWFIENEEQEYVQTLKSSKGGPGSAVSPYPTFNPSSDV (SEQ ID NO:8; Annexin A1 2-48 V24L), optionally C-terminally amidated.

11. The liquid pharmaceutical formulation according to any of the preceding claims, wherein pH is 8,3 said formulation comprising

    i) 5 mg/mL of Annexin A1 2-48 V24L (SEQ ID NO: 8), optionally C-terminally amidated,
    ii) 4 % (w/w) mannitol,
    iii) 1 % (w/w) sucrose,
    iv) 0,01 % (w/w) Polysorbate-80, and
    v) 10 mM glycyl-glycine.

12. A process of preparing a lyophilized pharmaceutical formulation, said process comprising the steps of

    a. providing the liquid pharmaceutical formulation according to any of the preceding claims, and
    b. lyophilizing said liquid pharmaceutical formulation of step a.

13. A method of making a reconstituted solution comprising an Annexin A1 N-terminal-peptide, said method comprising

    a. providing a lyophilized pharmaceutical formulation according to claim 12, and
    b. dissolving said lyophilized pharmaceutical formulation in a solvent.

14. The liquid pharmaceutical formulation according to any of claims 1 to 11 for use as a medicament.

15. The liquid pharmaceutical formulation according to any of claims 1 to 11 for use in the treatment of an ischemic condition and/or an inflammatory condition.

## Patentansprüche

1. Flüssige pharmazeutische Formulierung mit einem pH-Wert > 6,0; die Formulierung umfassend

    a. ein Annexin-A1- (AnxA1-) N-terminales Peptid, das eine Aminosäuresequenz aufweist, ausgewählt aus der Gruppe, bestehend aus

    AMVSEFLKQAWFIENEEQEYVQTVKSSKGGPGSAVSPYPTFNPSSDVAA (SEQ ID NO:4; Annexin-A1 2-50),
    AMVSEFLKQAWFIENEEQEYVQTVKSSKGGPGSAVSPYPTFNPSSDV (SEQ ID NO:5; Annexin-A1 2-48),
    AMVSEFLKQAWFIENEEQEYVQTVKSSKGGPGSAVSPYPTFNPSS (SEQ ID NO:6; Annexin-A1 2-46),
    AMVSEFLKQAWFIENEEQEYVQTLKSSKGGPGSAVSPYPTFNPSSDVAA (SEQ ID NO:7; Annexin-A1 2-50 V24L),
    AMVSEFLKQAWFIENEEQEYVQTLKSSKGGPGSAVSPYPTFNPSSDV (SEQ ID NO:8; Annexin-A1 2-48 V24L), und
    AMVSEFLKQAWFIENEEQEYVQTLKSSKGGPGSAVSPYPTFNPSS (SEQ ID NO:9; Annexin-A1 2-46 V24L),
    und eine Variante einer der SEQ ID NO:4-9, umfassend 1 bis 6 einzelne Aminosäuresubstitutionen, wobei die Variante ein Ligand und/oder Agonist von einem oder mehreren von Formylpeptid-Rezeptor 1 (FPR1), Formylpeptid-Rezeptor 2 (FPR2) und Formylpeptid-Rezeptor 3 (FPR3) ist; und

    b. ein Lösungsmittel, ausgewählt aus der Gruppe, bestehend aus

    i. Wasser, wie Reinstwasser; und
    ii. eine Lösung, die eine Ionenstärke von 1 $\mu$M bis 50 mM aufweist,

wobei das Lösungsmittel einen oder mehrere Isotoniemodifier umfasst, ausgewählt aus einem Kohlenhydrat und/oder einem Zuckeralkohol.

2. Flüssige pharmazeutische Formulierung mit einem pH-Wert ≥ 8; die Formulierung umfassend

a. ein Annexin-A1- (AnxA1-) N-terminales Peptid, das eine Aminosäuresequenz aufweist, ausgewählt aus der Gruppe, bestehend aus

AMVSEFLKQAWFIENEEQEYVQTVKSSKGGPGSAVSPYPTFNPSSDVAA (SEQ ID NO:4; Annexin-A1 2-50),
AMVSEFLKQAWFIENEEQEYVQTVKSSKGGPGSAVSPYPTFNPSSDV (SEQ ID NO:5; Annexin-A1 2-48),
AMVSEFLKQAWFIENEEQEYVQTVKSSKGGPGSAVSPYPTFNPSS (SEQ ID NO:6; Annexin-A1 2-46),
AMVSEFLKQAWFIENEEQEYVQTLKSSKGGPGSAVSPYPTFNPSSDVAA (SEQ ID NO:7; Annexin-A1 2-50 V24L),
AMVSEFLKQAWFIENEEQEYVQTLKSSKGGPGSAVSPYPTFNPSSDV (SEQ ID NO:8; Annexin-A1 2-48 V24L), und
AMVSEFLKQAWFIENEEQEYVQTLKSSKGGPGSAVSPYPTFNPSS (SEQ ID NO:9; Annexin-A1 2-46 V24L),
und eine Variante einer der SEQ ID NO:4-9, umfassend 1 bis 6 einzelne Aminosäuresubstitutionen, wobei die Variante ein Ligand und/oder Agonist von einem oder mehreren von Formylpeptid-Rezeptor 1 (FPR1), Formylpeptid-Rezeptor 2 (FPR2) und Formylpeptid-Rezeptor 3 (FPR3) ist; und

b. ein Lösungsmittel, wobei das Lösungsmittel eine Lösung ist, die eine Ionenstärke von 1 $\mu$M bis 50 mM aufweist.

3. Flüssige pharmazeutische Formulierung nach Anspruch 1, wobei der pH-Wert $\geq$ 7,4, wie z. B. pH-Wert $\geq$ 7,5, wie z. B. pH-Wert $\geq$ 7,6, wie z. B. pH-Wert $\geq$ 7,7, wie z. B. pH-Wert $\geq$ 7,8, wie z. B. pH-Wert $\geq$ 7,9, wie z. B. pH-Wert $\geq$ 8,0, wie z. B. pH-Wert $\geq$ 8,3 beträgt.

4. Flüssige pharmazeutische Formulierung nach einem der vorhergehenden Ansprüche, wobei der pH-Wert 8 bis 8,5 beträgt.

5. Flüssige pharmazeutische Formulierung nach einem der vorhergehenden Ansprüche, wobei das Annexin-A1-N-terminale Peptid eine wässrige Löslichkeit von mindestens 0,1 mg/ml, wie z. B. mindestens 0,5 mg/ml, wie z. B. mindestens 1,0 mg/ml, wie z. B. mindestens 1,5 mg/ml, wie z. B. mindestens 2,0 mg/ml, wie z. B. mindestens 2,5 mg/ml, wie z. B. mindestens 3,0 mg/ml, wie z. B. mindestens 3,5 mg/ml, wie z. B. mindestens 4,0 mg/ml, wie z. B. mindestens 5,5 mg/ml, wie z. B. mindestens 5,0 mg/ml, wie z. B. mindestens 7,5 mg/ml, wie z. B. mindestens 10,0 mg/ml aufweist,
und/oder die Formulierung eine Konzentration des Annexin-A1-N-terminalen Peptids von 0,1 bis 10 mg/ml, wie z. B. 0,1 bis 0,5 mg/ml, wie z. B. 0,5 bis 1 mg/ml, wie z. B. 1 bis 2 mg/ml, wie z. B. 2 bis 3 mg/ml, wie z. B. 3 bis 4 mg/ml, wie z. B. 4 bis 5 mg/ml, wie z. B. 5 bis 6 mg/ml, wie z. B. 6 bis 7 mg/ml, wie z. B. 7 bis 8 mg/ml, wie z. B. 8 bis 9 mg/ml, wie z. B. 9 bis 10 mg/ml aufweist.

6. Flüssige pharmazeutische Formulierung nach einem der Ansprüche 2-5, wobei das Lösungsmittel einen oder mehrere Isotoniemodifier wie z. B. ein Kohlenhydrat und/oder einen Zuckeralkohol umfasst.

7. Flüssige pharmazeutische Formulierung nach einem der vorhergehenden Ansprüche, wobei der Isotoniemodifier ausgewählt ist aus der Gruppe, bestehend aus Pentose-Monosacchariden, Hexose-Monosacchariden, Disacchariden und Trisacchariden,
und/oder wobei der Isotoniemodifier ausgewählt ist aus der Gruppe, bestehend aus:
Saccharose, Trehalose, Mannose, Ribose, Maltose, Glukose, Laktose, Galaktose, Arabinose, Mannit, Sorbit, Inosit, Glycerin, Xylit, Propylenglykol, Polyethylenglykole (PEG) und Polypropylen/Ethylenglykol-Copolymer.

8. Flüssige pharmazeutische Formulierung nach einem der vorhergehenden Ansprüche, wobei die Lösung Saccharose und/oder Mannit umfasst,
optional wobei die Lösung ca. 4 Gew./Vol.-% Mannit und/oder ca. 1 % Saccharose umfasst, wie z. B 3 bis 5 Gew./Vol.-% Mannit und/oder 0,5 bis 2 Gew./Vol.-% Saccharose umfasst, wie z. B 0 bis 8 Gew./Vol.-% Mannit und/oder 0 bis 5 Gew./Vol.-% Saccharose umfasst.

9. Flüssige pharmazeutische Formulierung nach einem der vorhergehenden Ansprüche, ferner umfassend einen oder mehrere Puffer,
wie z. B. ein oder mehrere Puffer ausgewählt aus der Gruppe, bestehend aus einem Acetatpuffer, TRIS, aminhaltigen Puffern, aminosäurebasierten Puffern wie z. B. Lysin, Hydroxylysin, Histidin und Glycylglycin, Nichtphosphatpuffern, Bicarbonatpuffern, Polysorbat-80 (z. B. Tween 80), HEPES und Borat.

**10.** Flüssige pharmazeutische Formulierung nach einem der vorhergehenden Ansprüche, wobei das Annexin-A1-N-terminale Peptid die Aminosäuresequenz AMVSEFLKQAWFIENEEQEYVQTLKSSKGGPGSAVSPYPTFNPSSDV (SEQ ID NO:8; Annexin-A1 2-48 V24L) aufweist, optional C-terminal amidiert.

**11.** Flüssige pharmazeutische Formulierung nach einem der vorhergehenden Ansprüche, wobei der pH-Wert 8,3 beträgt, die Formulierung umfassend

> i) 5 mg/mL Annexin-A1 2-48 V24L (SEQ ID NO: 8), optional C-terminal amidiert,
> ii) 4 Gew.-% Mannit,
> iii) 1 Gew.-% Saccharose,
> iv) 0,01 Gew.-% Polysorbat-80, und
> v) 10 mM Glycylglycin.

**12.** Prozess zum Zubereiten einer lyophilisierten pharmazeutischen Formulierung, wobei der Prozess die folgenden Schritte umfasst

> a. Bereitstellen der flüssigen pharmazeutischen Formulierung nach einem der vorhergehenden Ansprüche, und
> b. Lyophilisieren der flüssigen pharmazeutischen Formulierung aus Schritt a.

**13.** Verfahren zum Herstellen einer rekonstituierten Lösung, umfassend ein Annexin-A1-N-terminales Peptid, das Verfahren umfassend

> a. Bereitstellen einer lyophilisierten pharmazeutischen Formulierung nach Anspruch 12, und
> b. Auflösen der lyophilisierten pharmazeutischen Formulierung in einem Lösungsmittel.

**14.** Flüssige pharmazeutische Formulierung nach einem der Ansprüche 1 bis 11 zur Verwendung als Medikament.

**15.** Flüssige pharmazeutische Formulierung nach einem der Ansprüche 1 bis 11 zur Verwendung bei der Behandlung einer ischämischen und/oder entzündlichen Bedingung.

**Revendications**

**1.** Formulation pharmaceutique liquide de pH > 6,0 ; ladite formulation comprenant

> a. un peptide N-terminal d'annexine A1 (AnxA1) ayant une séquence d'acides aminés choisie dans un groupe constitué de
>
> > AMVSEFLKQAWFIENEEQEYVQTVKSSKGGPGSAVSPYPTFNPSSDVAA (SEQ ID NO:4 ; annexine A1 2-50),
> > AMVSEFLKQAWFIENEEQEYVQTVKSSKGGPGSAVSPYPTFNPSSDV (SEQ ID NO:5 ; annexine A1 2-48),
> > AMVSEFLKQAWFIENEEQEYVQTVKSSKGGPGSAVSPYPTFNPSS (SEQ ID NO:6 ; annexine A1 2-46),
> > AMVSEFLKQAWFIENEEQEYVQTLKSSKGGPGSAVSPYPTFNPSSDVAA (SEQ ID NO:7 ; annexine A1 2-50 V24L),
> > AMVSEFLKQAWFIENEEQEYVQTLKSSKGGPGSAVSPYPTFNPSSDV (SEQ ID NO:8 ; annexine A1 2-48 V24L), et
> > AMVSEFLKQAWFIENEEQEYVQTLKSSKGGPGSAVSPYPTFNPSS (SEQ ID NO:9 ; annexine A1 2-46 V24L),
> > et un variant de l'une quelconque des SEQ ID NO: 4 à 9 comprenant 1 à 6 substitutions d'acides aminés individuelles, dans laquelle ledit variant est un ligand et/ou un agoniste d'un ou de plusieurs parmi le récepteur de peptide de formyle 1 (FPR1), le récepteur de peptide de formyle 2 (FPR2) et le récepteur de peptide de formyle 3 (FPR3) ; et
>
> b. un solvant choisi dans un groupe constitué
>
> > i. de l'eau, telle que de l'eau ultrapure ; et
> > ii. d'une solution ayant une force ionique de 1 $\mu$M à 50 mM,

dans laquelle ledit solvant comprend un ou plusieurs modificateurs d'isotonicité choisis parmi un glucide et/ou un alcool de sucre.

2. Formulation pharmaceutique liquide de pH ≥ 8 ; ladite formulation comprenant

   a. un peptide N-terminal d'annexine A1 (AnxA1) ayant une séquence d'acides aminés choisie dans un groupe constitué de

   AMVSEFLKQAWFIENEEQEYVQTVKSSKGGPGSAVSPYPTFNPSSDVAA (SEQ ID NO:4 ; annexine A1 2-50),
   AMVSEFLKQAWFIENEEQEYVQTVKSSKGGPGSAVSPYPTFNPSSDV (SEQ ID NO:5 ; annexine A1 2-48),
   AMVSEFLKQAWFIENEEQEYVQTVKSSKGGPGSAVSPYPTFNPSS (SEQ ID NO:6 ; annexine A1 2-46),
   AMVSEFLKQAWFIENEEQEYVQTLKSSKGGPGSAVSPYPTFNPSSDVAA (SEQ ID NO:7 ; annexine A1 2-50 V24L),
   AMVSEFLKQAWFIENEEQEYVQTLKSSKGGPGSAVSPYPTFNPSSDV (SEQ ID NO:8 ; annexine A1 2-48 V24L), et
   AMVSEFLKQAWFIENEEQEYVQTLKSSKGGPGSAVSPYPTFNPSS (SEQ ID NO:9 ; annexine A1 2-46 V24L),
   et un variant de l'une quelconque des SEQ ID NO: 4 à 9 comprenant 1 à 6 substitutions d'acides aminés individuelles, dans laquelle ledit variant est un ligand et/ou un agoniste d'un ou de plusieurs parmi le récepteur de peptide de formyle 1 (FPR1), le récepteur de peptide de formyle 2 (FPR2) et le récepteur de peptide de formyle 3 (FPR3) ; et

   b. un solvant, dans laquelle ledit solvant est une solution ayant une force ionique de 1 μM à 50 mM.

3. Formulation pharmaceutique liquide selon la revendication 1, dans laquelle pH ≥ 7,4, tel que pH ≥ 7,5, tel que pH ≥ 7,6, tel que pH ≥ 7,7, tel que pH ≥ 7,8, tel que pH ≥ 7,9, tel que pH ≥ 8,0, tel que pH ≥ 8,3.

4. Formulation pharmaceutique liquide selon l'une quelconque des revendications précédentes, dans laquelle le pH est de 8 à 8,5.

5. Formulation pharmaceutique liquide selon l'une quelconque des revendications précédentes, dans laquelle le peptide N-terminal d'annexine A1 a une solubilité aqueuse d'au moins 0,1 mg/ml, telle qu'au moins 0,5 mg/ml, telle qu'au moins 1,0 mg/ml, telle qu'au moins 1,5 mg/ml, telle qu'au moins 2,0 mg/ml, telle qu'au moins 2,5 mg/ml, telle qu'au moins 3,0 mg/ml, telle qu'au moins 3,5 mg/ml, telle qu'au moins 4,0 mg/ml, telle qu'au moins 5,5 mg/ml, telle qu'au moins 5,0 mg/ml, telle qu'au moins 7,5 mg/ml, telle qu'au moins 10,0 mg/ml,
   et/ou ladite formulation ayant une concentration dudit peptide N-terminal d'annexine A1 de 0,1 à 10 mg/ml, telle que 0,1 à 0,5 mg/ml, telle que 0,5 à 1 mg/ml, telle que 1 à 2 mg/ml, telle que 2 à 3 mg/ml, telle que 3 à 4 mg/ml, telle que 4 à 5 mg/ml, telle que 5 à 6 mg/ml, telle que 6 à 7 mg/ml, telle que 7 à 8 mg/ml, telle que 8 à 9 mg/ml, telle que 9 à 10 mg/ml.

6. Formulation pharmaceutique liquide selon l'une quelconque des revendications 2 à 5, dans laquelle ledit solvant comprend un ou plusieurs modificateurs d'isotonicité, tels qu'un glucide et/ou un alcool de sucre.

7. Formulation pharmaceutique liquide selon l'une quelconque des revendications précédentes, dans laquelle ledit modificateur d'isotonicité est choisi dans un groupe constitué des monosaccharides pentoses, des monosaccharides hexoses, des disaccharides et des trisaccharides,
   et/ou dans laquelle ledit modificateur d'isotonicité est choisi dans un groupe constitué de : saccharose, tréhalose, mannose, ribose, maltose, glucose, lactose, galactose, arabinose, mannitol, sorbitol, inositol, glycérol, xylitol, propylène glycol, polyéthylène glycols (PEG) et copolymère polypropylène/éthylène glycol.

8. Formulation pharmaceutique liquide selon l'une quelconque des revendications précédentes, dans laquelle la solution comprend du saccharose et/ou du mannitol,
   éventuellement dans laquelle la solution comprend environ 4 % (p/v) de mannitol et/ou environ 1 % de saccharose, par exemple comprend 3 à 5 % (p/v) de mannitol et/ou 0,5 à 2 % (p/v) de saccharose, par exemple comprend 0 à 8 % (p/v) de mannitol et/ou 0 à 5 % (p/v) de saccharose.

9. Formulation pharmaceutique liquide selon l'une quelconque des revendications précédentes comprenant également un ou plusieurs tampons,

tel qu'un ou plusieurs tampons choisis dans un groupe constitué d'un tampon acétate, TRIS, des tampons contenant des amines, des tampons à base d'acides aminés tels que la lysine, l'hydroxy-lysine, l'histidine et la glycyl-glycine, des tampons non phosphates, des tampons bicarbonates, du polysorbate 80 (par exemple Tween 80), de l'HEPES et du borate.

10. Formulation pharmaceutique liquide selon l'une quelconque des revendications précédentes, dans laquelle ledit peptide N-terminal d'annexine A1 a la séquence d'acides aminés AMVSEFLKQAWFIENEEQEYVQTLKSSKGGPGSAVSPYPTFNPSSDV (SEQ ID NO: 8 ; annexine A1 2-48 V24L), éventuellement amidée en C-terminal.

11. Formulation pharmaceutique liquide selon l'une quelconque des revendications précédentes, dans laquelle le pH est de 8,3, ladite formulation comprenant

    i) 5 mg/ml d'annexine A1 2-48 V24L (SEQ ID NO: 8), éventuellement amidée en C-terminal,
    ii) 4 % (p/p) de mannitol,
    iii) 1 % (p/p) de saccharose,
    iv) 0,01 % (p/p) de polysorbate 80, et
    v) 10 mM de glycyl-glycine.

12. Processus de préparation d'une formulation pharmaceutique lyophilisée, ledit processus comprenant les étapes de

    a. fourniture de la formulation pharmaceutique liquide selon l'une quelconque des revendications précédentes, et
    b. lyophilisation de ladite formulation pharmaceutique liquide de l'étape a.

13. Procédé de fabrication d'une solution reconstituée comprenant un peptide N-terminal d'annexine A1, ledit procédé comprenant

    a. la fourniture d'une formulation pharmaceutique lyophilisée selon la revendication 12, et
    b. la dissolution de ladite formulation pharmaceutique lyophilisée dans un solvant.

14. Formulation pharmaceutique liquide selon l'une quelconque des revendications 1 à 11 pour une utilisation comme médicament.

15. Formulation pharmaceutique liquide selon l'une quelconque des revendications 1 à 11 pour une utilisation dans le traitement d'un état ischémique et/ou d'un état inflammatoire.

Fig. 1

Fig. 2

Fig. 3

Fig. 4

| pH 7.01 | pH 6.1-4.2 | pH 3.5 | pH 2.6-2.0 |
|---|---|---|---|
| | | | |

Fig. 5

Fig. 6

Fig. 7

AxnA1 purity after 3 month at 40°C

Fig. 8

Fig. 9

EP 4 200 319 B1

46

Fig. 10

Fig. 11

**EP 4 200 319 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012174397 A